(19) Europäisches Patentamt
European Patent Office
Office européen des brevets

(11) EP 2 747 776 B1

(12) # EUROPEAN PATENT SPECIFICATION

(45) Date of publication and mention of the grant of the patent:
**11.07.2018 Bulletin 2018/28**

(21) Application number: **12758914.1**

(22) Date of filing: **24.08.2012**

(51) Int Cl.:
*A61K 38/57* (2006.01)
*A61K 38/10* (2006.01)
*C07K 16/38* (2006.01)
*G01N 33/86* (2006.01)
*A61P 7/04* (2006.01)

(86) International application number:
**PCT/NL2012/050581**

(87) International publication number:
**WO 2013/028070 (28.02.2013 Gazette 2013/09)**

(54) **COMPOUNDS FOR USE IN BOOSTING COAGULATION**

VERBINDUNGEN ZUR VERSTÄRKUNG DER KOAGULATION

COMPOSÉS DESTINÉS À ÊTRE UTILISÉS POUR FAVORISER LA COAGULATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.08.2011 US 201161527140 P**

(43) Date of publication of application:
**02.07.2014 Bulletin 2014/27**

(73) Proprietor: **UMC Utrecht Holding B.V.**
**3584 CM Utrecht (NL)**

(72) Inventors:
- **HACK, Cornelis Erik**
  **NL-1111 BV Diemen (NL)**
- **YILDIZ, Cafer**
  **NL-6846 LT Arnhem (NL)**

(74) Representative: **Nederlandsch Octrooibureau**
**P.O. Box 29720**
**2502 LS The Hague (NL)**

(56) References cited:
**WO-A1-2009/013251**

- **BJORK I ET AL: "CONVERSION OF ANTITHROMBIN FROM AN INHIBITOR OF THROMBIN TO A SUBSTRATE WITH REDUCED HEPARIN AFFINITY AND ENHANCED CONFORMATIONAL STABILITY BY BINDING OF A TETRADECAPEPTIDE CORRESPONDING TO THE P-1 TO P-14 REGION OF THE PUTATIVE REACTIVE BOND LOOP OF THE INHIBITOR", JOURNAL OF BIOLOGICAL CHEMISTRY, vol. 267, no. 3, 1992, pages 1976-1982, XP055054309, ISSN: 0021-9258**
- **MUSZBEK LASZLO ET AL: "Antithrombin deficiency and its laboratory diagnosis", CLINICAL CHEMISTRY AND LABORATORY MEDICINE, vol. 48, no. Suppl. 1, December 2010 (2010-12), pages S67-S78, XP009167217, ISSN: 1434-6621**
- **KUMMER J A ET AL: "Production, characterization, and use of serpin antibodies", METHODS : A COMPANION TO METHODS IN ENZYMOLOGY, ACADEMIC PRESS INC., NEW YORK, NY, US, vol. 32, no. 2, 1 February 2004 (2004-02-01), pages 141-149, XP004481665, ISSN: 1046-2023, DOI: 10.1016/S1046-2023(03)00205-6 cited in the application**
- **MAYER STEPHAN A ET AL: "Recombinant activated factor VII for acute intracerebral hemorrhage", NEW ENGLAND JOURNAL OF MEDICINE, THE, MASSACHUSETTS MEDICAL SOCIETY, WALTHAM, MA, US, vol. 352, no. 8, 24 February 2005 (2005-02-24), pages 777-785, XP002449328, ISSN: 0028-4793, DOI: 10.1056/NEJMOA042991 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 747 776 B1

## Description

### Field of the invention

The invention relates to the field of medicine, in particular the invention relates to blood clotting factors, inhibitors thereof and their use for hemostasis. More specifically a method is disclosed to boost thrombin activity to stimulate blood clotting. The invention can be used to treat or prevent excessive bleeding, for example due to a bleeding disorder such as hemophilia, or due to surgery, trauma, or to the use of anticoagulant drugs, or to stop an internal bleeding.

### Background of the invention

Clotting of blood, coagulation, is a delicate process which should be switched on immediately in case of vascular injury, and switched off when the vasculature is intact. Pathology results when this process is regulated incorrectly: a bleeding disorder or extensive bleeding results when coagulation is switched on too late or too weakly, a thrombotic disease ensues when coagulation is switched on too early or too strongly. This invention discloses a novel method to stimulate coagulation and hence can be applied in diseases or clinical conditions characterized by (excessive) bleeding. These conditions include hemophilia A or B, bleeding due to surgery or trauma, as well as internal bleeding such as hemorrhagic stroke and gastro-intestinal bleeding, or bleeding to the use of anticoagulant drugs such as warfarin or heparin.

Clotting of blood comprises a humoral and a cellular response. The former leads to the conversion of soluble fibrinogen into insoluble fibrin, the latter to the formation of a platelet plug. Both responses are intertwingled. For example, the enzyme thrombin, which is formed during the humoral response, is a potent activator of platelets, whereas the membranes of activated platelets serve to assemble clotting factors complexes, which are pivotal for an efficient coagulation process. Activated platelet plugs, fibrin threads and included red and white blood cells together constitute a blood clot. This patent deals with a novel method to boost the humoral response of blood clotting. The humoral coagulation response will therefore be discussed first.

The humoral coagulation response is mediated by a series of proteins, the clotting factors, which circulate as inactive cofactors and pro-enzymes. Together these factors constitute the coagulation system. Activation of coagulation comprises a process consisting of a sequential activation of inactive pro-enzymes and cofactors in a cascade-like fashion. At each step, a newly formed clotting protease will catalyze the activation of the next factor. Activation is started upon contact of blood with the membrane protein tissue factor (TF), which is normally not expressed by endothelial cells, but is abundantly present on sub-endothelial cells such as smooth muscle cells. Hence, disruption of the endothelial cell layer will expose blood to this constitutively expressed TF, which triggers coagulation. During activation of the coagulation system several enzyme-cofactor complexes are formed, which assemble on the membranes of cells, particularly endothelial cells and activated platelets. Among these enzyme-cofactor complexes is that of TF and activated factor VII (TF-FVIIa), in which FVIIa has proteolytic activity which can activate factors X and IX (FX and FIX). Activated FX (FXa) assembles with activated factor V (FVa) to form the prothrombinase complex, which in its turn activates prothrombin into the active protease thrombin. TF-FVIIa can also convert factor IX into FIXa which assembles with activated factor VIII (FVIIIa) to generate the FVIIIa-FXIa complex. The active protease in this complex is FIXa, which, just as FVIIa, can activate FX.

Upon contact with appropriate stimuli, e.g. TF, activation of the coagulation system is initiated by the TF-FVIIa complex and propagated by the action of the FVIIIa-FIXa complex and amplified by the activation of FXI (see Figure 1).

Under physiological conditions most hemostatic responses need this FIX- and FVIII-dependent amplification to ensure sufficient activation of FX (and hence thrombin generation). This need for FVIII and IX is dramatically shown by the clinical manifestations of genetic deficiencies of these proteins: hemophilia A or B, which are severe bleeding disorders and result from a deficiency of FVIII or FIX, respectively.

Hemophilia is a X-linked congenital bleeding disorder generally resulting from a deficiency of clotting FVIII (Hemophilia A) or FIX (Hemophilia B) (Mannucci et al., N Engl J Med 2001;344:1773-1779). The frequency of the disease is about one in 10,000 male births. The prevalence of hemophilia A is about 5-6 times that of hemophilia B, which also is a X-linked genetic deficiency. Clinically hemophilia is categorized into mild, moderate and severe hemophilia, depending on the severity of the bleeding manifestations. Persons with severe hemophilia often have a history of easy bruising in early childhood, spontaneous bleeding (particularly into the joints and soft tissue), and excessive bleeding following trauma or surgery. Patients with mild hemophilia hardly suffer from spontaneous bleeding episodes, and mainly have excessive trauma or surgery-induced bleeding. Appropriate clotting tests will confirm the diagnosis and reveal whether a patient suffers from hemophilia A or B. Severe hemophilia A is characterized by very low or absent functional FVIII levels (<1% of normal), whereas in case of mild hemophilia levels of functional FVIII are 5-15% of normal.

On demand and prophylactic therapy for hemophilia patients typically consists of replacement therapy, which is based on the complementation of the patient's defective coagulation system with the lacking coagulation factor. Thus,

hemophilia A and B patients are infused with FVIII and FIX concentrates, respectively, to treat or to prevent bleeding episodes. Hemophilia patients treated with preparations of the deficient factor are, however, at risk to develop neutralizing antibodies (inhibitors) to the missing factor. The missing clotting factor is not endogenous for these patients and consequently their immune system has not become tolerant to it. Formation of neutralizing antibodies occurs in 15-30% of severe hemophilia A patients (Goudemond et al., Blood 2006; 107:46-51; Gouw et al., Blood. 2007;109:4693-4697) and 1-3% of hemophilia B patients. Particularly patients with severe gene defects that lead to FVIII plasma levels <1% of normal, such as gene deletion or inversion, nonsense and frameshift mutations, may develop inhibitors since their immune system has not become tolerant to FVIII. When circulating levels of inhibitors are high, patients require very high amounts of the deficient factor to overcome the neutralizing effects of the inhibitors. Such high dose FVIII therapy is very expensive. As an alternative, FVIII bypass therapy has been developed. The principle underlying these treatments is that the defect in the FVIII/FIX complex is bypassed by the use of activated forms of FVII, IX and X. Examples of such FVIII bypass therapies are activated prothrombin-complex concentrates and recombinant FVIIa (J Astermark et al., Blood. 2007;109:546-551; HR Roberts et al., Blood 2004;104:3858-3864; WK Hoots, Blood 2007;109:395-396). A disadvantage of these FVIII-bypass therapies is that they are only suitable for on demand therapy.

The only recombinant FVIII-bypass therapy licensed is recombinant FVIIa (rFVIIa; NovoSeven®, NovoNordisk, Copenhagen, Denmark). rFVIIa stimulates hemostasis by binding, directly or in complex with tissue factor, to negatively charged phospholipids exposed on the surface of activated platelets, thereby targeting activation of FX to the site of injury. Alternatively, its effect may be due to an increase in the ratio of FVIIa to FVII. rFVIIa stops bleeding in 70-75% of the patients with inhibitors. A limitation of FVIII-bypass therapy with rFVIIa or activated prothrombin-complex is that it is expensive and not applicable as prophylactic therapy due to its short half-life of 2-3 hours. Yet, prophylactic treatment of hemophilia prevents hemophilic arthropathy and improves quality of life (Manco-Johnson et al., N Engl J Med 2007; ;357:535-44). Thus, there is an unmet medical need for prophylactic FVIII-bypass therapy of hemophiliacs with FVIII inhibitors. It is an object of the present invention to provide such a therapy.

Several other clinical conditions are accompanied by excessive or unwanted bleeding such as surgery induced bleeding, bleeding due to war or traffic accidents, internal bleedings such as hemorrhagic stroke and gastro-intestinal bleeding, and bleeding associated with the use of anticoagulants such as warfarin. These conditions can be treated with hemostatic agents that are applied locally such as fibrin sealants, or with systemically administered rFVIIa. Treatment of these conditions with rFVIIa, however, has a risk for triggering thrombo-embolic processes leading to serious side effects (O'Connel et al., JAMA. 2006;295:293-298; Levi et al., N Engl J Med 2010;363:1791-1800). This thrombo-embolic risk possibly has to do with that rFVIIa is an active clotting protease. Infusion of rFVIIa in the circulation thus results in the presence of an active clotting protease everywhere in the circulation. In addition, rFVIIa is difficult to titrate due to the lack of a simple assay to monitor its pharmacodynamic effects.

It is an object of the present invention to provide for novel compounds and methods for the treatment and prevention of excessive bleeding due to surgery, accidents and other conditions. In contrast to therapy with rFVIIa, the novel compounds and methods of the invention can be used as a prophylactic therapy, as well as on demand therapy, and their therapeutically required dosage can be titrated and monitored with a simple and convenient bedside assay.

## Summary of the invention

The invention provides a method for treatment and prevention of excessive bleeding, comprising of administration of an inhibitor of antithrombin III (AT) to decrease levels of functional AT to a bleeding patient or a patient at risk for bleeding. Thus, in a first aspect, the invention relates to an inhibitor of antithrombin III (AT) for use in the treatment and prevention of bleeding: i) in a subject suffering from an acquired or genetic bleeding disorder (hypocoagulability); or, ii) in a subject having a clinical condition characterised by excessive bleeding, wherein the bleeding is not due to the use of anti-coagulants; wherein the inhibitor is selected from the group consisting of: a) a peptide or peptidomimetic that mimicks the amino acid sequence of RSL residues P1-P14; b) an aptamer that specifically binds to antithrombin III; and, c) an antibody or antibody fragment that specifically binds to antithrombin III, and wherein the inhibitor, when added to pooled plasma from healthy volunteers, causes a decrease of functional antithrombin III levels by at least 20% as measured with a chromogenic assays for functional antithrombin III using factor Xa or thrombin. This medical use is surprising and novel as AT inhibitors have never been described as a therapeutic method to treat or prevent bleeding in patients. The invention can be applied in hemophilia, surgery, trauma or other conditions in which excessive bleeding occurs such as the use of anticoagulants, and also in case of internal bleeding such as hemorrhagic stroke or gastro-intestinal bleeding such as oesophageal bleeding due to varices. The medical use disclosed by the present invention can also be used to prevent bleeding in patients at risk for bleeding, for example in haemophilia patients to prevent bleeding in joints, muscle and/or soft tissues in a hemophilia patient. Bleeding can be prevented in such a patient by treatment with an AT inhibitor.

In certain embodiments, the AT inhibitor is a molecule that binds to AT and inhibits the function of AT or increases the clearance of AT in humans or in an animal. In other embodiments of the invention this binding molecule is a peptide

or an antibody or an antibody fragment. Also other molecules such as chemical compounds, that bind to AT and inhibit its function, are in the scope of the present invention.

In certain embodiments of the invention the AT inhibitor is a monoclonal antibody, human, humanized or from an animal species, or fragments thereof, that bind to AT and inhibit the activity of AT.

In other embodiments of the invention the AT inhibitor is a monoclonal antibody, human, humanized or from an animal species, or fragments thereof, that bind to AT and decrease the levels of AT in humans or in an animal.

In other embodiments of the invention the AT inhibitor is polyclonal antibodies, or fragments thereof, that inhibit the activity of AT.

In other embodiments of the invention the AT inhibitor is a polyclonal antibody, human, humanized or from an animal species, or fragments thereof, that bind to AT and decrease the levels of AT in humans or in an animal.

In other embodiments of the invention the AT inhibitor is a non-antibody molecule such as an aptamer, peptide or chemical compound that binds to AT and inhibits the activity of AT.

In other embodiments of the invention the AT inhibitor is a non-antibody molecule such as an aptamer, protein, peptide or chemical compound that binds to AT and decreases the levels of AT in humans or in an animal.

In some embodiments of the invention the AT inhibitor binds to and blocks sites on AT involved in the interaction of AT with its target proteases. In other embodiments of the invention the AT inhibitor binds to sites on AT thereby preventing the increase of functional activity of AT by heparin or other glycosaminoglycans.

In a preferred embodiment of the present invention the AT inhibitor prevents insertion of the reactive site loop of AT into the central β-sheet of AT, which causes proteolytic inactivation of AT by its target proteases.

In another embodiment of the invention the AT inhibitor is administered to treat or prevent bleeding in a hemophilia patient with FVIII deficiency who may or may not have neutralizing antibodies against FVIII. In other embodiments, the hemophilia patient is deficient in FIX activity and may or may not have neutralizing antibodies against FIX.

In another embodiment of the invention the AT inhibitor is administered to treat or prevent bleeding in a hemophilia patient with FXI deficiency. In other embodiments, AT inhibitor is given to treat or prevent bleeding in a patient with a clotting factor deficiency other than that of FVIII, FIX or FXI.

In an embodiment, the AT inhibitor according to the invention reduces or prevents the possibility of generating inhibitors to FVIII in a hemophilia A patient, thereby decreasing the need for FVIII. In another embodiment, the AT inhibitor according to the invention reduces or prevents the possibility of generating inhibitors to FIX in a hemophilia B patient.

In an embodiment, the AT inhibitor according to the invention administered to non-hemophiliacs who suffers from (excessive) bleeding, wherein the bleeding is not due to the use of anti-coagulants. Thus, in one embodiment, the invention relates to an inhibitor of AT for use in the prevention or treatment of a clinical condition characterised by excessive bleeding, such as e.g. surgery, trauma and internal bleeding.

According to the embodiments of the invention described above, a hemostatic amount of AT inhibitor is administered to said patient. Said amount preferably is an effective hemostatic amount.

In certain embodiments, the dosage of AT inhibitor to be administered to a hemophilia patient is calculated from an in vitro assay which is used to establish the concentration of said AT inhibitor that can compensate the clotting factor that is deficient in said plasma, comprising: a) adding to plasma from a hemophilia patient a dilution of tissue factor and $Ca^{2+}$ at concentrations where the clotting time is dependent on addition of the clotting factor (e.g. FVIII or FIX) that is deficient in said plasma; b) measuring fibrin generation or thrombin generation in hemophilic plasma in the absence of said clotting factor; c) measuring fibrin generation or thrombin generation in haemophilic plasma in the presence of the deficient clotting factor added to achieve concentrations of 0.01 to 1 U/ml, whereby 1 U/ml is the amount of factor present in pooled normal human plasma; and d) measuring fibrin generation or thrombin generation (as well as AT levels) in haemophiliac plasma in the absence of said clotting factor but in the presence of various concentrations of AT inhibitor. The desired level of functional AT to be achieved by administration of AT inhibitor can be estimated by comparison of thrombin generation and fibrin formation at different AT inhibitor levels with those observed at different levels of the deficient factor.

In another embodiment, the dosage of AT inhibitor to be administered to a haemophilia patient with inhibitors is calculated from an in vitro assay which is used to establish the concentration of said AT inhibitor that can compensate the clotting factor that is deficient in said plasma, comprising: a) adding to plasma from a hemophilia patient with inhibitors of FVIII or IX a dilution of tissue factor and $Ca^{2+}$ at concentrations where the clotting time would be dependent on addition of the clotting factor (e.g. FVIII or FIX) in haemophilic plasma with inhibitors; b) measuring fibrin generation or thrombin generation in the haemophilic plasma with inhibitors; and c) measuring fibrin generation or thrombin generation in pooled normal plasma. The dose of AT-inhibitor that normalizes thrombin generation and fibrin formation can be selected as the target level for in vivo administration. The dose of AT inhibitor to be used in vivo is then monitored by administering AT inhibitor to the patient while measuring functional AT level in the circulation.

In another embodiment the disclosure provides a method for estimating the capacity of AT inhibitor to stimulate fibrin formation and thrombin generation and activity in a plasma from a non-hemophilic patient bleeding because of surgery, trauma, the use of anticoagulants or because of another reason, comprising: a) providing plasma of that patient

with a dilution of tissue factor; b) measuring fibrin generation or thrombin generation in that plasma; c) measuring fibrin generation or thrombin generation in the presence of a dose of AT inhibitor that causes a drop of AT activity between 0 and 1 U/ml (0 and 100% of normal, respectively); and d) measuring fibrin generation or thrombin generation in said plasma in absence or presence of AT inhibitor, to establish the capacity of AT inhibitor to increase fibrin formation of thrombin generation in said plasma.

In another embodiment the dose of AT inhibitor to be used in vivo is monitored by administering AT inhibitor to the patient while measuring functional AT level in the circulation.

In one aspect, the invention provides a method for treating a bleeding patient or a patient at risk for bleeding, comprising administering to the patient an AT inhibitor to obtain a plasma concentration of functional AT below 80% of normal, preferably between 20 and 80%, preferably between 20 and 60% of normal, more preferably between 40 and 60% of normal, more preferably of about 50% of normal. Thus, preferably, the inhibitor is administered in an amount that decreases the functional activity of AT to at least 90, 80, 50, 20, 10, 5, 1, 0.5, 0.2, 0.1% of the normal AT level, whereby the normal AT is preferably herein understood the level in pooled plasma from healthy volunteers.

In certain embodiments the invention provides a method for treating a non-hemophilic patient having a bleeding episode by administering to the patient AT inhibitor to obtain a plasma concentration of functional AT below 80% of normal, preferably between 20 and 80%, preferably between 20 and 60% of normal, more preferably between 40 and 60% of normal, more preferably of about 50% of normal. The dose of AT inhibitor administered is monitored using an assay for plasma levels of functional AT.

In another aspect, the invention provides a method for treating a patient having a FVIII deficiency suboptimal hemostatic level of FVIII administered to the patient in combination with AT inhibitor, to yield an effective hemostatic amount of the mix. In certain embodiments, FVIII, in combination with AT inhibitor, is administered at a level sufficiently low to not provoke an immune response in the patient to FVIII.

## Detailed description of the invention

Excessive activation of coagulation in vivo is regulated by several inhibitors. A main inhibitor is antithrombin III (AT), which belongs to the protein family of serine proteinase *in*hibitors or serpins. Until now nobody has shown or suggested that inhibition of AT constitutes a therapeutic approach for boosting coagulation. The present invention discloses the unexpected finding that inhibition of AT constitutes a therapeutic option for patients with haemophilia or other bleeding disorders. By administering an inhibitor of AT, e.g. an inhibiting antibody, to a patient suffering from or at risk for bleeding, AT levels are decreased allowing more thrombin to be generated and prolonging the activity of thrombin, which leads to the generation of more fibrin and more efficient blood clot formation.

### *Rationale of the invention*

AT inhibits several clotting proteinases particularly thrombin and FXa, and also FVIIa, FIXa and FXIa. It has been estimated that about 80% of the amount of thrombin generated in vivo is neutralized by AT. Decreased levels of functional AT, for example due to a genetic heterozygous deficiency, results in less inhibition of thrombin, FXa, FIXa, FXIa and FVIIa, and hence in more fibrin generation. Notably, at decreasing AT levels not only the half-life of thrombin activity is prolonged but also more thrombin molecules are generated as the half-life of FXa, the clotting protease that generates thrombin from prothrombin, is also prolonged. Prolonged FXA activity increases the number of prothrombin molecules converted into thrombin per FXa molecule. Thus, the procoagulant effects of lowering AT levels is due to a longer half-life of thrombin as well as to an increased amount of thrombin generated. This is in contrast to all other forms of FVIII bypass such as therapy with rFVIIa, which only increases the generation of thrombin but has no effect on the half life of thrombin. Inhibition of AT in patients with or at risk for bleeding constitutes a novel therapeutic principle that has never been described in the literature.

The primary defect in hemophilia is the absence of amplification of thrombin generation via the FVIII/FIX loop (see Figure 1). However, some thrombin generation occurs in absence of the FVIII/FIX loop since the pathway of direct activation of FX by the FVIIa/TF complex is intact. In plasma thrombin is inhibited for 60-90% by AT. Furthermore, also the activities of FXa and FIXa are inhibited by AT. One embodiment of the present invention is the administration of a therapeutically effective dose of an AT inhibitor to reduce inhibition of FXa and thrombin by AT. As a consequence the half-life of both proteases is prolonged resulting in a larger number of prothrombin molecules converted into thrombin per molecule of FXa, and a larger number of fibrinogen molecules converted into fibrin per thrombin molecule. Thus, by decreasing functional levels of AT, more thrombin is generated and the half-life of the activity of thrombin is prolonged. This compensates the decreased generation of thrombin which occurs in hemophilia as a consequence of a defective function of the FVIII/FIX loop. Notably, this dual effect of AT inhibition, which is increasing the number of thrombin molecules generated as well as prolonging the activity of thrombin, is unique to the concept of AT inhibition and is not shared with any of the other FVIII bypass procedures. This makes AT inhibition in principle the most potent FVIII bypass

approach.

Inhibition of AT in itself is not a trigger for activation of coagulation, since activation requires active proteases. Interaction of TF with FVII(a) and the subsequent activation of FIX and FX generates active proteases at the site of TF expression (see figure 1). Thus, though due to the lower level of functional AT clotting occurs more easily in presence of AT inhibitors, this clot formation mainly, if not exclusively, will be limited to places in the body where TF is exposed to blood, for example in injured vessel walls. This is in contrast to treatment with recombinant FVIIa, which is administered as a an active protease and will become available throughout the whole circulatory system.

*Aspects of the invention*

Thus in a first aspect the disclosure relates to an inhibitor of AT for use as a medicament. The inhibitor preferably is an inhibitor of AT as herein defined below.

In a second aspect, the invention relates to an inhibitor of AT for use in the treatment and prevention of bleeding in a subject suffering from an acquired or genetic bleeding disorder (hypocoagulability) or in a subject having a clinical condition characterized by excessive bleeding, wherein the bleeding is not due to the use of anti-coagulants; wherein the inhibitor is selected from the group consisting of: a) a peptide or peptidomimetic that mimicks the amino acid sequence of RSL residues P1-P14; b) an aptamer that specifically binds to antithrombin III; and, c) an antibody or antibody fragment that specifically binds to antithrombin III, and wherein the inhibitor, when added to pooled plasma from healthy volunteers, causes a decrease of functional antithrombin III levels by at least 20% as measured with a chromogenic assays for functional antithrombin III using factor Xa or thrombin.. Alternatively in this aspect, the invention pertains to the use of an inhibitor of AT for the manufacture of a medicament for the treatment and prevention of bleeding in a subject suffering from an acquired or genetic bleeding disorder (hypocoagulability) or in a subject having a clinical condition characterized by excessive bleeding. In this aspect, the inhibitor of AT preferably is an inhibitor as herein defined below. Likewise, the treatment and prevention of bleeding in a subject suffering from an acquired or genetic bleeding disorder (hypocoagulability) or in a subject having a clinical condition characterized by excessive bleeding are preferably as herein defined below.

In a further aspect, the disclosure relates to a method for treating or reducing the risk of bleeding in a subject suffering from an acquired or genetic bleeding disorder (hypocoagulability) or in a subject having a clinical condition characterized by excessive bleeding, the method comprising the step of administering to the subject an effective amount of an inhibitor of AT. The inhibitor of AT preferably is an inhibitor as herein defined below. Likewise, the acquired or genetic bleeding disorder (hypocoagulability) and the clinical condition characterized by excessive bleeding are preferably as herein described below.

In another aspect, the disclosure relates to a method for identifying as compound as an inhibitor of AT herein defined below.

*Inhibitors of antithrombin III (AT)*

Antithrombin III (AT) is an 58 kDa glycoprotein belonging to the protein family of serpins (serine protease inhibitors). The mature protein consists of 432 amino acids, and has 4 glycosylation sites. AT is produced in the liver, in parenchymal cells. Its plasma concentration is about 0.1-0.15 mg/ml or 3.5 $\mu$M. AT disappears from the circulation with an apparent half-life of 2-3 days. Antigenic and activity levels are expressed as a percentage of the normal level or as U/ml, 1 U being the amount in pooled human plasma. AT is expressed as both an $\alpha$-form and a $\beta$-form. $\alpha$-AT represents 90% of AT and is glycosylated at all four positions. While comprising only 10% of AT, $\beta$-AT, has a higher affinity for heparin, is not glycosylated at one position (N135), and exerts an overall larger anticoagulant effect than $\alpha$-AT.

AT is the most important physiological inhibitor of the coagulation pathway. As its name implies, AT inhibits thrombin. In addition, AT is capable of inhibiting all of the other proteolytic coagulation factors (e.g. FIXa, FXa and FXIa). Its main anticoagulant activity is, however, due to the regulation of FXa, FIXa and thrombin. It is estimated that AT provides 50-80% or up to 80% of the inhibitory effect against thrombin in vivo.

The action of AT is potentiated by glycosaminoglycans such as heparin and heparan-sulphate, which can increase the functional activity of AT by 2-3 logs. Heparin consists of a protein backbone carrying a number of sulphated disaccharides. Crude preparations contain heparin molecules with a molecular weight ranging from 3 to 40 kDa. A unique pentasaccharide sequence in heparin is responsible for high affinity binding to AT. In vivo, heparan sulphate on the endothelium, and heparin released from endothelium-associated mast cell granules are probably main sources for cofactors of AT.

Heparin uses two distinct mechanisms to accelerate protease inhibition by AT. One mechanism is that it makes the reactive site loop (RSL; in Figure 2 this loop is called the reactive center loop (RCL), see Figure 2) more accessible to target proteases, which mechanism contributes to the enhanced inhibitory activity towards FIXa and FXa. Another mechanism is that heparin provides a template for AT and its target proteases, particularly thrombin. In addition to its

anticoagulant activity, AT has been shown to have anti-inflammatory and anti-angiogenic functions as well. These will not be discussed here.

The physiological importance of AT has been shown in knockout mice with complete AT deficiency. These mice die in utero from thrombosis and haemorrhage (Ishiguro et al., J Clin Invest.2000;106:873-878). Also in humans total deficiency of AT has presumably lethal effects since a complete AT deficiency has not been documented in humans whereas heterozygous deficiency occurs in 1 to 2,000-5,000 persons. Heterozygous AT deficient persons have an increased risk for thrombo-embolic disorders (PS McLean et al., Drugs 2007;67: 1429-1440).

The effects of the inhibition of clotting proteases by AT has been investigated in a system with purified proteins at the normal plasma concentration (van 't Veer et al., J Biol Chem.1997;272:4367-4377). The presence of AT at normal levels did not affect the initiation phase of thrombin generation when the reaction was initiated with FVIIa/TF, nor did AT affect FV activation. The rate of thrombin formation in the presence of AT was reduced to 30% that of the uninhibited reaction, and the amount of thrombin formed was rapidly quenced subsequent to its generation. The present invention discloses that lowering AT levels can be used as a therapeutic principle in patients with severe bleeding or at risk for severe bleeding.

AT belongs to the protein superfamily of serpins (serine-protease inhibitors) which are widely found in nature occurring f/e in animals, plants, bacteria and viruses (see for review: Ruby HP Law et al., Genome Biology 2006;7:216.1-216.11). Most serpins have well-known names which mostly refers to the function which has led to their discovery. Although for some serpins like AT the traditional name more or less covers their main biological functions, for some other serpins their traditional name is misleading in this respect. For example, α1-antitrypsin and α1-antichymotrypsin have been discovered because of their potency to inhibit the digestive enzymes trypsin and chymotrypsin, respectively, but their main function in vivo is to inhibit neutrophilic proteases, elastase and protease 3, and cathepsin G, respectively. Serpins are classified into 16 clades (A-P). The systematic name of each serpin is, SERPINXy, where X is the clade and y is the number within the clade. According to this classification AT is serpinC1. Humans have genes coding for 36 serpins. Most serpins serve as protease inhibitor although some have adopted different functions.

The basic structure of serpins is made up of about 400 amino acids, some have large N-, C-terminal or internal insertion loops. Most serpins have posttranslational modifications such glycosylation, sulfation, phosphorylation and oxidation to alter their function. Serpins share a highly conserved 3-D structure comprised of a bundle of 9 α-helices (A-I) and a β-sandwich composed of three β-sheets (A-C) (see Figure 2). Another typical feature of serpins is the reactive site loop (RSL) which protrudes from the body of the protein and is presented to proteases as a kind of bait. The RSL is typically composed of 20 amino acids running from P17 at the N-terminus to P3' at the C-terminal end (according to this nomenclature P1-P1' constitute the scissile bond, which can be cleaved by proteases). In the normal native state of a serpin, β-sheet A, or the central β-sheet, is composed of five strands and the RSL (bridging the C-terminus of strand 5A to the N-terminus of strand 1C) is exposed. Remarkably, thermodynamic stability of this conformation is not optimal, incorporation of the RSL into the central β-sheet yields a more stable conformation. This stable conformation is achieved either upon proteolytic cleavage of the RSL by a (non-target) protease, in which case the protease is released from the serpin before the insertion of the RSL into the central β-sheet is completed, or during complex formation with a target protease, in which case the protease is still covalently bound to the amino acid residue on the PI-position when the insertion is completed. As a consequence of this insertion the active site serine is slightly pulled out from its position in the active site of the protease thereby preventing that the protease can be released from the P1-residue. In the latter case covalently linked complexes between a conformationally changed serpin and its target protease are left.

The interaction of a serpin with a target protease is stoichiometric. The reaction can be described as:

$$P + S \leftrightarrow PS \rightarrow PS^* \rightarrow P + S^*$$

P = protease; S = serpin; S* = serpin with altered, stable conformation

Upon recognition of the sequence of the RSL (RCL in Figure 3) as a substrate by the protease, a reversible complex (also known as the Michaelis complex) is formed in which the conformation of the serpin is still native (S). In next stage the protease starts to cleave the peptidyl bond between the amino acid residues at the P1- and P1'-position (in case of a non-target proteases other peptidyl bonds in the RSL may be cleaved as well), and the N-terminal portion of RSL inserts into the central β-sheet of the serpin as this yields its lowest energy conformation. When insertion is completed before the protease has been released from P1, the active site of the protease is distorted immediately interrupting the proteolytic reaction of the protease (conformation c in Figure 3).

This results in a stable complex in which the active site of the protease is trapped covalently by the RSL of the serpin, and in which the serpin has adapted the stable conformation (S*). When the insertion is not yet completed when the protease has completed its hydrolytic reaction, the protease is released as an active enzyme and an inactivated serpin in which the cleaved RSL is inserted into the central β-sheet (conformation d in Figure 3). While the obligate RSL-active site contacts contribute significantly to the formation of the Michaelis complexes, other sites of the molecule are

also involved.

In case of AT, covalent complexes between the serpin and the proteases thrombin, FXa and FIXa, and to some extent also FXIa and FXIIa can be formed. The efficiency of inhibition of these proteases can be described in kinetic constants, particularly second order rate constants. These constants are given in Table 1.

Table 1: Inhibition constants of AT with several target proteases and the influence of glycosaminoglycans on these (after: Rau et al., J.Thromb.Haemost 2007;5 (Suppl. 1):102-115.

| Target protease | Cofactor | Second order rate $k_2$ ($M^{-1}s^{-1}$) |
|---|---|---|
| Thrombin | -- | $7.5 \times 10^3$, $1 \times 10^4$ |
| | UFH | $2 \times 10^7$, $4.7 \times 10^7$ |
| | LMWH | $5.3 \times 10^6$ |
| | Pentasaccharide | $2 \times 10^4$ |
| Factor Xa | -- | $2.5 \times 10^3$, $6 \times 10^3$ |
| | UFH | $5 \times 10^6$, $6.6 \times 10^6$ |
| | LMWH | $1.3 \times 10^6$ |
| | Pentasaccharide | $7.5 \times 10^5$ |
| Factor IXa | -- | $1.3 \times 10^2$, $5 \times 10^2$ |
| | UFH | $8 \times 10^6$, $1.75 \times 10^6$ |
| | LMWH | $3.7 \times 10^5$ |
| UFH: unfractionated heparin; LMWH: low molecular weight heparin | | |

As is clear from Table 1, the inhibitory activity of AT is enhanced in the presence of glycosaminoglycans such as heparin, or synthetic pentasaccharides. The enhanced inhibition of proteases by AT in the presence of heparin results from two distinct mechanisms. It expels the N-terminus of the RSL from the central β-sheet A. This "liberation" of the RSL explains the enhanced inhibition of FIXa and FXa, but not that of thrombin. The enhanced inhibition of thrombin results from a so-called template mechanism which implies that heparin serves as a template to bind both thrombin and AT in an optimal position to allow maximal inhibition of thrombin by AT.

In principle any pharmaceutically acceptable inhibitor of AT can be used in the present invention. A suitable inhibitor of AT for use in the present invention preferably is an inhibitor, which, when added to plasma (preferably human plasma, more preferably pooled plasma from healthy volunteers), causes a decrease of the activity of AT as preferably measured with factor Xa, thrombin, or any other protease interacting with AT, such as e.g. factor IXa. Preferably, the inhibitor decreases the activity of AT to at least 90, 80, 50, 20, 10, 5, 1, 0.5, 0.2, 0.1% of the normal AT level. The normal AT is herein understood the level in pooled plasma from healthy volunteers. Alternatively, the inhibitor decreases the activity of AT to at least 90, 80, 50, 20, 10, 5, 1, 0.5, 0.2, 0.1% of the AT level prior to addition of the inhibitor.

The inhibition of AT is preferably measured by comparing the effect of AT in plasma with and without the inhibitor when added to an proteolytic enzyme that is normally inhibited by AT, such as e.g. thrombin, factor Xa or factor IXa , from human or bovine origin. Thus, preferably, the inhibitor, when added to plasma (preferably human plasma, more preferably pooled plasma from healthy volunteers), prevents a reduction in activity of at least one of thrombin, factor Xa and factor IIa by more than 10, 20, 50, 80, 90, 95, 99, 99.5, 99.8, 99.9% by the plasma when added to at least one of (isolated) thrombin, (isolated) factor Xa, and (isolated) factor IIa, as compared to the reduction in activity of at least one of thrombin and factor Xa by the same plasma without the inhibitor. Preferably, the activity of thrombin and/or factor Xa is determined in an assay using a chromogenic substrate as e.g. described at http://www.practical-haemostasis.com/Thrombophilia%20Tests/at assays.html or in Beeck et al., Blood Coagulation Fibrinolysis 2000;11:127-35. A suitable chromogenic substrate for factor Xa is e.g. S-2765, Z-D-Arg-Gly-Arg-pNA.2HCl having the formula:

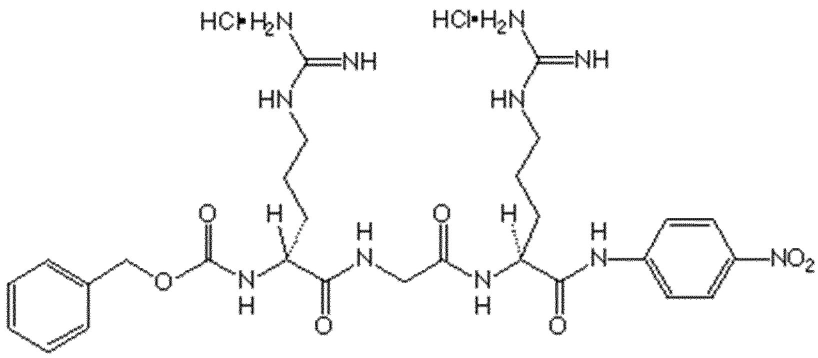

Assays for AT activity are further described herein under *"Determination of functional AT levels to assess the inhibition of AT".*

A preferred inhibitor of AT for use in the present invention is an inhibitor is selected from the group consisting of: (a) a compound that can insert into the central β-sheet of antithrombin III; (b) an aptamer that specifically binds to antithrombin III and prevents or slows down the insertion of the RSL-loop into the central β-sheet of antithrombin III; and, (c) an antibody or antibody fragment that specifically binds to AT. Such inhibitors are further described herein below.

*Peptide-inhibitors of AT*

In one embodiment, a class of AT inhibitors that can be used in the present invention, e.g. to enhance thrombin formation and thrombin activity in patients suffering from bleeding is based on the observation that peptides that mimick the sequence of RSL residues P1-P14 can insert into the central β-sheet of AT. Insertion of the peptide into the central β-sheet prevents the insertion of the RSL of the molecule itself. As insertion of the RSL into the central β-sheet is essential for the inhibitory function of the serpin (see Figures 2 and 3), AT incubated with the RSL-peptide is converted into a substrate for its target proteases such as thrombin. In other words, upon incubation of AT the peptide mimicking the RSL amino acid sequence of AT will bind and insert into the central β-sheet of AT and turn AT into a substrate for its target proteases (see Figure 4). The effects of peptides mimicking the sequence of P1-P14 of the RSL of AT has been described by I Bjork et al., J.Biol.Chem.1992;267:1976-1982. Thus, a preferred inhibitor of AT according to this embodiment is a peptide or peptidomimetic that mimicks the amino acid sequence of RSL residues P1-P14.

A preferred sequence of this peptide is:

$NH_2$**-Ser-Glu-Ala-Ala-Ala-Ser-Thr-Ala-Val-Val-Ile-Ala-Gly-Arg-**COOH

Notably, nobody until now has claimed or suggested the therapeutic use of peptides mimicking the sequence of AT RSL as a therapy for patients with excessive bleeding. Such peptides can be a peptide with the indicated sequence, or a peptide with the same sequence but linked to another protein sequence, or to one or several polyethylene glycol (PEG) molecules or other molecules to improve the pharmacokinetic properties. Also variants of the indicated sequence capable of binding to AT are considered to fall within the scope of this invention.

Furthermore, also chemical or other compounds such as aptamers capable of binding to AT and inactivating AT by preventing or slowing down the insertion of the RSL-loop and thereby decreasing the inhibitory activity towards thrombin and other clotting proteases are considered to fall within the scope of this patent.

*Antibody-inhibitors of AT*

In another embodiment, the AT inhibitor for use in the present invention comprises polyclonal and monoclonal antibodies (human, murine, humanized murine, other animal species such as camelid) against AT, or fragments thereof. The specific examples herein demonstrate that polyclonal antibodies against human AT upon addition to plasma fully block AT activity in plasma.

Studies on monoclonal auto-antibodies that neutralize and inactivate C1-inhibitor, which also is a serpin and shares structural homology with AT, and that cause acquired C1-inhibitor deficiency, have revealed that these antibodies recognize epitopes located near to the RSL sequence around P10' (S He et al., J.Immunol. 1996;154: 2009-2013). These antibodies render C1-inhibitor a substrate for its target proteases (S He et al., Molecular Medicine 4: 119-128, 1998), probably by hampering the insertion of the RSL of C1-inhibitor into the central beta-sheet (see also right panel of Figure 4). Antibodies or fragments thereof against the homologous sequences of AT are used to inactivate AT upon interaction with target proteases. Antibodies or fragments thereof against the RSL sequence of AT around P10 also impair insertion of RSL into the central β-sheet and render the serpin into a substrate when interacting with a target protease. Indeed, one antibody recognizing an epitope involving the amino acids at position P3-P8 of the RSL of AT,

has been shown to render AT into a substrate when incubated with thrombin (S Asakura et al., J Biol Chem 1990;265:5135-6). Moreover, other monoclonal antibodies that inhibit AT without further specification of the underlying mechanisms of inhibition, have been described in the literature (P Herion et al., Blood 1985;65:1201-7; S Knoller et al., Eur J Bioch 1989;180:319-27). However, the therapeutic potential of such antibodies for the treatment or prevention of bleeding has never been considered. The present invention discloses that such antibodies can be used to stop and prevent bleeding in hemophiliacs or patients with bleeding episodes because of other reasons. All antibodies, fully human, humanized or from animal species, de-immunized or not, or fragments thereof, that inhibit AT by interfering with the insertion of the RSL of AT into the central beta-sheet, fall within the scope of this invention.

Thus, a preferred inhibitor according to the invention is an antibody or antibody fragment specifically binds to an epitope in the RSL sequence of AT, and, preferably, impairs insertion of the RSL into the central β-sheet of AT. More preferably thereby, the epitope in the RSL sequence of AT comprises one or more of the RSL residues PI-P14, preferably the epitope comprises RSL residues around P10, RSL residues P5 - P10 or RSL residues P3 - P8.

Antibodies against the sequences of the RSL-peptides mentioned above are made by immunization with peptides covering these sequences either or not linked to a carrier protein. Keyhole Limpet Hemocyanin (KLH) conjugated peptides are routinely used as antigens to generate antibody against peptide sequences. Other carrier proteins, however, such as bovine serum albumin (BSA), can also be used. Moreover, mutants of the indicated peptide sequence also can be used for immunization, as long as the resulting antibodies bind to AT.

A preferred strategy to make AT-inhibiting antibodies is to immunize a suitable host with KLH to which peptides having the amino acid sequence of RSL of AT have been coupled. The peptides can be purchased from commercial or non-commercial suppliers, or be made according to procedures well known in the art. Also commercially available peptide synthesiser machines can be used for this purpose. Peptides are coupled to KLH via well established methods, for example by introduction of a C-terminal cysteine in the sequence. Another method uses glutaraldehyde as a conjugation reagent. Several commercial companies offer production of KLH-peptide conjugates as a service to clients. Such KLH-peptides conjugates can then be used for immunization of a suitable host.

In a preferred embodiment KLH is used as a carrier protein, peptides covering the RSL sequence (see above) or peptides covering the sequences P5'-P10 of the RSL of AT, are conjugated to KLH via introduction of an additional cysteine residue at the C-terminus.

A variety of immunization protocols may be employed, and may include intravenous, subcutaneous, or intraperitoneal injection, followed by one or more boosts. A suitable adjuvant is Freund's adjuvant. A suitable immunization procedure is hyperimmunization with KLH-peptide at a concentration that, dependent on the host animal, may be in the range of 10 to 500 microgram, mixed with Freund's complete adjuvant. The mix is injected subcutaneously. Injections are repeated 2 to 5 times using the same KLH-peptide preparation mixed with Freund's incomplete adjuvant. Blood samples are taken from the animal 1 week after the $3^{rd}$, $4^{th}$, $5^{th}$ injection, and screened for the presence of antibodies against AT.

In a preferred embodiment of this invention monoclonal murine antibodies against AT are made by using hybridoma technology. Monoclonal antibodies can be produced in various other ways with techniques well understood by those having ordinary skill in the art. Details of these techniques are described in (Antibodies: A Laboratory Manual, Harlow et al., Cold Spring Harbor Publications, p. 726,1988), or are described by (Campbell, A.M. "Monoclonal Antibody Technology Techniques in Biochemistry and Molecular Biology," Elsevier Science Publishers, Amsterdam, The Netherlands, 1984) or by (St. Groth et al., J. Immunol. Methods 35:1-21, 1980).

Hybridomas that secrete antibody against human AT can be identified by assaying culture supernatants, etcetera, for the presence of antibody. The preferred screening procedure comprises two sequential steps, the first being identification of hybridomas that secrete mAb against AT, the second being determination of the ability of the mAb to inhibit the functional activity of AT.

An ELISA is used to screen hybridoma supernatants for the presence of anti-AT monoclonal antibodies. This ELISA can be performed as follows. Purified human plasma AT (or recombinant human AT) is used for coating (2 μg/ml in phosphate buffered saline, pH 7.4 [PBS]; 100 ul/well). Residual non-specific binding sites are then blocked by 30 minutes incubation at room temperature with PBS/0.1% (w/v) Tween 20 (PBS-T) containing 0.2% (w/v) gelatin (PBS-TG). Then, after a washing procedure (5 times with PBS-T), the plates are incubated for 60 minutes at 37°C with 20 μl of hybridoma supernatant together with 80 μl of PBS-TG. Finally, bound murine antibodies are detected by incubation with peroxidase-conjugated polyclonal goat anti-mouse immunoglobulin antibodies for 120 minutes at 37°C. Finally, the plates are washed with distilled water (5 times), and developed with 3,5,3',5'-tetramethyl benzidine.

Alternatively, anti-AT antibodies are measured with a radioimmunoassay procedure, for example using a similar procedure as described for detection of anti-C3 antibodies by (Hack et al., J Immunol. 1988; 141:1602-9).

To assess the effect of the antibody against AT on the functional activity of AT, positive hybridomas are subcloned via limiting dilution and grown up. Next, antibodies are purified from hybridoma culture supernatant by protein G-affinity chromatography. Purified antibody is then incubated with purified AT or with human plasma, after which the functional activity of AT is measured in a chromogenic assay as described hereinbelow.

Any antibodies or fragments thereof that inhibit AT via a different mechanism than interfering with the RSL insertion, are also considered to fall within the scope of this invention. These antibodies can be human, humanized, murine, deimmunized monoclonal antibodies, antibodies from other animal species such as camels, or synthetic, or fragments thereof. The key features of the antibodies or antibody fragments that are within the scope of the present invention, is that they bind to AT and inhibit the functional activity of AT. These antibodies block one or more functionally important sites on AT resulting in a decreased functional activity of AT.

Antibodies that inhibit AT in general can be obtained by immunization of animals with functional AT. Antibodies (either polyclonal by collection of plasma and purification of AT-antibodies, or monoclonal antibodies made via the hybridoma technology with native AT as antigen) are then screened with assays using AT as antigen. All positive antibodies are then tested for inhibitory activities towards AT by incubating purified AT with the antibodies after which the functional activity of AT is tested using a chromogenic assay as described hereinbelow. Alternatively, the antibodies can be screened by adding them to plasma and testing the plasma in clotting assays such as the prothrombin time (PT) and activated prothromboplastin time (aPTT) or other. Moreover, the antibody can be added to normal human plasma, after which the functional activity of AT is measured using a chromogenic assay as described below. Inhibiting antibodies will decrease the functional activity of AT in these assays.

In addition, antibodies of fragments thereof, that bind to AT and cause a decrease of AT in vivo by enhancing the clearance of AT, or decrease AT activity in vivo by other mechanisms, are considered to fall within the scope of the present invention.

*Other inhibitors of AT*

Peptides, antibodies and other molecules that bind to AT and inhibit AT via different mechanisms than interfering with the insertion of the RSL in the central beta-sheet, are also within the scope of this invention. For example, these peptides or compounds such as aptamers may bind to other functional sites on AT such as sites involved in the interaction with target proteases. Any molecule able to inhibit the functional activity of AT, preferably *in vivo,* is considered to fall within the scope of the invention. Likewise, any molecule able to decrease the functional activity of AT, preferably *in vivo* is considered to fall within the scope of the invention.

*Determination of functional AT levels to assess the inhibition of AT*

The effect of AT inhibitors in plasma can be assessed with an assay that measures the functional activity of AT in plasma or in other (biological) fluids. Functional activity of AT can be assessed based on its inhibitory activity towards thrombin. Such assays are commercially available (f/e Actichrome ATIII, American Diagnostica, Greenwich, CT, USA; STA Stachrome, Diagnostica Stago Inc, Parsippany, NJ, USA). The basis of these assays is that thrombin can convert suitable chromogenic substrates (f/e S-2238, Haemochrom Diagnostica, Molndal, Sweden). Chromogenic substrates are peptides that react with proteolytic enzymes under the formation of color. They are made synthetically by several companies and are designed to possess a selectivity similar to that of the natural substrate for the enzyme. Attached to the peptide part of the chromogenic substrate is a chemical group, p-nitroanilide, which upon release after the enzyme cleavage gives rise to a yellow color. The color change can be followed spectrophotometrically and is proportional to the proteolytic activity. In case of AT measurements a given amount of thrombin is incubated with plasma in step 1, during which step functional AT binds to and inactivates part of the thrombin. Residual thrombin activity is then measured with a chromogenic substrate in step 2 of the assay. This residual thrombin activity is inversely proportional to the AT concentration. To ensure optimal quantification the incubation of thrombin with plasma occurs in the presence of heparin which optimizes the activity of AT. Furthermore, bovine thrombin is often used to increase further the specificity of the assay, since bovine thrombin, in contrast to human thrombin, hardly interacts with another plasma inhibitor, heparin cofactor II. Also human or bovine FXa in combination with a chromogenic substrate can be used to measure AT activity levels.

Using this chromogenic assay the level of AT inhibition by AT inhibitors can be easily measured both in vitro as well as in vitro. In vitro this is achieved by incubating a certain volume of plasma, for example X ml, with a certain amount of AT inhibitor dissolved in, for example, 0.1X ml saline or buffer. The mixture is then incubated at 37°C, after which AT activity in the mixture is measured with a chromogenic assay for AT as described above. The value obtained is then compared with the value obtained with a sample containing thrombin and saline, rather than plasma, which is equal to 100% inhibition of functional AT, and that obtained with plasma to which no AT inhibitors have been added, which equals 0% inhibition. The amount of AT inhibition in the plasma sample incubated with AT inhibitor is than compared with the 0% and the 100% inhibition controls and expressed as % inhibition of AT. The plasma sample incubated with AT inhibitor can be a normal plasma or plasma from a bleeding patient or a patient at risk for bleeding, for example a hemophiliac. In vivo inhibition of AT by AT inhibitors administered to a patient can be assessed by measuring circulating AT levels upon administration of AT inhibitors using the chromogenic assays described in this paragraph.

*Clinical application of the invention*

AT inhibitors can be applied for prophylactic or on demand treatment of bleeding disorders. Thus in one embodiment, the invention relates to an inhibitor of AT for use in the treatment and prevention of bleeding in a subject suffering from bleeding disorder (hypocoagulability). The bleeding disorder can be an acquired or genetic bleeding disorder. In a preferred embodiment, the bleeding disorder is haemophilia, e.g. haemophilia A or B. More preferably, the inhibitor of AT is used in treating a hemophiliac subject with neutralizing antibodies to factor VIII or factor IX. In such instances the inhibitor is preferably used as prophylactic factor VIII or factor IX bypass therapy.

One application of the present invention is acute and prophylactic treatment of hemophiliacs with inhibitors (neutralizing antibodies to FVIII of IX). The severity of bleeding manifestations in hemophilia generally correlates with the clotting factor level. Severe hemophiliacs have negligible amounts of FVIII (<1% of normal) and may bleed spontaneously from many sites. Most frequent sites however are the joints (70%-80%) particularly knees and elbows, and muscle/soft tissue (10%-20%). Other major bleeds (5%-10%), and sometimes central nervous system bleeding (< 5%) also occur. In addition, hemophiliacs may bleed excessively following surgical or other trauma. Prophylactic replacement of clotting factor in hemophilia, which aims at keeping circulating levels of the deficient factor above 1% at all times to prevent spontaneous bleedings in joints and soft tissues, in contrast to on demand therapy largely prevents hemophilic arthropathy (MJ Manco-Johnson et al., N Engl J Med 2007; 357:535-44). In particular patients with very low or no FVIII are at risk of developing neutralizing antibodies when treated with exogenous FVIII, since their immune system considers the administered FVIII as a foreign antigen and is not tolerant to it. Indeed 20-30% of severe hemophiliacs develop such neutralizing antibodies (J Goudemond et al., Blood 2006;107:46-51; SC Gouw et al., Blood. 2007;109:4693-4697), which inhibit efficacy of administered FVIII and therefore are often referred to as FVIII inhibitors. At present, the only prophylactic therapy available for hemophiliacs is replacement therapy with the deficient factor. However, in case of a patient with inhibitors against FVIII, prophylactic therapy is virtually impossible as the administered FVIII is neutralized by the inhibitors or cleared rapidly from the circulation by binding of antibodies to FVIII. According to the present invention the defect of fibrin formation during coagulation in these patients can be restored by administration of an amount of AT inhibitor that decreases the functional activity of circulating AT, which increases fibrin formation due to diminished inhibition of factor Xa and to prolonged thrombin activity. The required degree of inhibition of AT can be monitored by measuring the in vivo level of functional AT upon administration of the AT inhibitor to a hemophilic patient, whereas the degree of inhibition required for effective hemostasis can be estimated based on in vitro experiments in which thrombin generation is induced by a low concentration of TF in plasma from the hemophilic patient in presence of various amounts of AT inhibitor.

In another embodiment, the invention relates to an inhibitor of AT for use in the prevention or treatment of a clinical condition characterised by excessive bleeding. A clinical condition characterised by excessive bleeding can e.g. be one or more of surgery, trauma and internal bleeding. Thus, the invention further provides a method for reducing or preventing blood loss in a trauma patient or a war victim, comprising administering an AT inhibitor to the patient. The invention also provides a method for reducing or preventing blood loss in a patient undergoing surgery, for example coronary bypass or other vascular surgery, comprising administering an AT inhibitor to the patient. The invention further provides a method for reducing or preventing brain damage in a patient suffering from an intracerebral bleeding, comprising administering an AT inhibitor to the patient. The invention also provides a method for reducing or preventing blood loss in a patient with an internal bleeding, for example from oesophageal varices or due to the use of non-steroidal anti-inflammatory drugs, comprising administering an AT inhibitor to the patient. The invention also provides a method for reducing or preventing blood loss in a patient with bleeding due to the use of anti-coagulants. The invention can be applied in surgery, trauma or other conditions in which excessive bleeding occurs such as the use of anticoagulants, and also in case of internal bleeding such as hemorrhagic stroke or gastro-intestinal bleeding such as oesophageal bleeding due to varices. The method disclosed by the present invention can also be used to prevent bleeding in patients at risk for bleeding, for example in haemophilia patients to prevent bleeding in joints, muscle and/or soft tissues in a hemophilia patient.

The inhibitor of antithrombin III when used in accordance with the invention in the treatment and prevention of bleeding in a subject suffering from an acquired or genetic bleeding disorder (hypocoagulability) or in a subject having a clinical condition characterised by excessive bleeding, is preferably used in a dosage that decreases the activity of AT to at least 90, 80, 50, 20, 10, 5, 1, 0.5, 0.2, 0.1% of the normal AT level, whereby the normal AT level is as herein defined above.

*Assessment of functional AT levels to guide the dose of AT inhibitor to be used in vivo*

The effect of AT inhibitors in plasma can be assessed using an assay that measures the functional activity of AT in plasma, as described hereinabove. The level of AT inhibition to be achieved in vivo can be estimated in vitro with plasma samples from patients with bleeding disorders. In case of a hemophilic plasma, the plasma supplemented with various concentrations of AT inhibitors may be FVIII-immune depleted plasma (Dade Behring, Marburg, Germany) or

plasma from a haemophilic patient. Thrombin formation in these plasma samples is then triggered by the addition of TF (for example Innovin, Dade Behring, B4212-50). TF concentrations are first selected to give a FVIII-dependent thrombin generation or fibrin formation in this FVIII-depleted or haemophilic plasma, which is achieved by incubating the plasma samples with 1 to 1000 to 1 to 80.000 dilutions of Innovin, in presence of different concentrations of FVIII ranging from 0.1% (0.001 U/ml) to 25% (0.25 U/ml) of the normal level mimicking severe to mild haemophilic conditions, respectively. Fibrin generation can be measured in time by use of the SpectraMax microtiterplate reader and Softmax pro software. Alternatively thrombin generation is assessed with chromogenic or fluorogenic substrates, f/e the Technothrombin TGA system provided by DiaPharma. A TF concentration is then selected that induces FVIII-dose-dependent thrombin- or fibrin generation in this system.

To assess the effect of AT inhibitor on fibrin formation or thrombin generation hemophilic or FVIII-depleted plasma samples are incubated with various amounts of AT inhibitor to decrease functional AT levels by 20, 40, 60, 80 or 100% as assessed according to the procedures described above. As controls, the plasma samples are supplemented with various concentrations of FVIII (f/e recombinant FVIII from Baxter (Recombinate®) or Bayer (Kogenate®). After mixing, the samples are tested for fibrin generation or thrombin generation upon addition of TF at a concentration selected as explained in the previous paragraph to yield FVIII-dependent thrombin generation and fibrin-formation. This thrombin generation and/or fibrin formation is then measured as described above. The enhanced (boosted) fibrin generation and/or thrombin generation observed in the plasma sample with decreasing levels of functional AT (increasing inhibition of AT), can be compared with the results obtained with FVIII. The desired level of AT inhibition to compensate the lack of FVIII can thus be selected to mimick conditions of moderate, mild or no hemophilia. The corresponding level of AT inhibitor to be achieved in plasma can then be calculated.

Co-administration of AT inhibitor with FVIII also can be considered. Such co-administration can decrease the need for FVIII and hence to decrease the risk of immunogenicity and the chance for inhibitor development. In patients, administering of Factor VIII can be diminished to much lower levels, for instance to levels sufficiently low to not provoke an immune response in the patient to FVIII. For instance, it is possible to administer to hemophilia patients an amount of FVIII to reach a concentration of 0.001-0.05, e.g. 0.01 U/ml plasma, and at the same time also treat the patient with AT inhibitor according to the present invention.

In addition to hemophilia, routine bleeding that fails conventional therapy may occur in a large number of other medical disorders. At present, rFVIIa, used off label, is the only treatment of these conditions. These conditions include intracranial hemorrhage, liver disease, bleeding after surgery such as cardiopulmonary bypass surgery, spinal surgery, prostate surgery and orthotopic liver transplantation, bleeding associated with trauma, bleeding associated with the use of anticoagulants such as warfarin, and other.

Intracerebral hemorrhage is one of the most disabling forms of stroke. More than one third of patients with this disorder die within one month after the onset of symptoms, the majority is severely debilitated, and only 20 percent regain functional independence. There is currently no effective treatment for intracerebral hemorrhage, though stimulation of blood clotting by administering recombinant FVIIa has shown promising results (SA Mayer et al., N.Engl.J.Med. 2005;352:777-85). The volume of the hematoma is a critical determinant of mortality and functional outcome after intracerebral hemorrhage, and early hematoma growth is an important cause of neurologic deterioration. rFVIIa has been shown to reduce this increase in volume. In a phase 3 study a favourable effect of rFVIIa on infarct size was again found, though the clinical consequences rFVIIa reduced growth of the hematoma but did not improve survival or functional outcome after intracerebral hemorrhage (SA Mayer et al., N.Engl.J.Med.2008;358:2127-37).

Patients with liver disease due to alcoholic or post-hepatitis cirrhosis or other diseases are at risk for severe bleeding due to decreased synthesis of clotting factors by the liver resulting in low circulating levels of clotting factors, and the presence of varices in the esophagus and stomach. These patients currently are treated with clotting factor concentrates or infusion of rFVIIa. Severe bleeding may also occur during liver transplantation and during partial hepatectomy in noncirrhotic patients due to decreased circulating levels of various clotting factors. Excessive hemorrhage may also in patients undergoing cardiac surgery. This increased bleeding risk is due to low plasma levels of clotting factors due to hemodilution as a consequence of the use of priming fluid for the extracorporeal oxygenator, the use of anticoagulants and mechanical issues of vascular grafts.

Anticoagulants are used for a number of thrombo-embolic diseases including atrial fibrillation, deep venous thrombosis, myocardial infarction and other. Patients treated with oral anticoagulant have lowered levels of functional vitamin K-dependent coagulation proteins and hence are at risk for hemorrhagic complications. The incidence of bleeding is around 0.6-0.7% per month at a therapeutic International Normalized Ratio (INR) and the incidence of major hemorrhagic events is substantial. Therefore, the ability to reverse the anti-vitamin K effect is important. In case of an emergency plasma or prothrombin complex concentrates are used, as well as recombinant FVIIa. As an alternative the present invention can be used. The advantage of the present invention is that the procoagulant effect can be titrated by measuring functional AT levels in a patient receiving AT-inhibitor.

In the clinical conditions described in the previous paragraph, as well as in other diseases with increased risk for bleeding or with ongoing bleeding, there is a medical need to prevent or to stop bleeding. To stop bleeding only

recombinant FVIIa or the plasma-derived activated prothrombin complex such as FEIBA are available (J Astermark et al., Blood 2007;109:546-551; HR Roberts et al., Blood 2004;104:3858-3864). None of these can be used to prevent bleeding. Furthermore there are no good in vitro assays that predict efficacy or side effects of these agents. The present invention provides an alternative to these procoagulants in that the effect of the treatment can be titrated, can be monitored, and can be used as prophilactic but also as on demand therapy. This invention consists of administration of AT-inhibitor to a patient at risk for bleeding or suffering from bleeding. Pharmacological control of the amount of AT-inhibitor to be administered can be achieved by measuring plasma levels of functional AT.

*Assessment of effective concentration of AT inhibitor to boost thrombin formation*

The target level of AT inhibition in vivo can be estimated in vitro with plasma samples from patients with bleeding disorders as is explained hereinabove. Upon addition of TF to induce coagulation, fibrin generation can be measured in time by use of the SpectraMax microtiterplate reader and Softmax pro software, whereas thrombin generation can be assessed with chromogenic or fluorogenic substrates, f/e the Technothrombin TGA system provided by DiaPharma.

Results of thrombin generation and fibrin formation in the plasma samples from patients containing varying amounts of AT inhibitor are compared to those obtained with plasma samples from normal controls. Based on the amount of AT inhibitor that is able to restore thrombin generation and fibrin formation into the range observed with the plasma samples from normal controls, as well as on bioavailability and pharmacokinetic properties, the dose of AT inhibitor to be administered is then calculated. The dose to be administered is confirmed by measuring circulating AT activity levels upon administration of AT inhibitors to the patients using the assay described hereinabove.

*Pharmaceutical compositions of AT inhibitors*

For administration to humans, the invention may employ pharmaceutical compositions comprising the AT inhibitor and a pharmaceutically acceptable carrier or excipient. In the present context, the term "Pharmaceutically acceptable" means that the carrier or excipient, at the dosages and concentrations employed, will not cause any unwanted or harmful effects in the patients to which they are administered. Such pharmaceutically acceptable carriers and excipients are well known in the art (see Remington’s Pharmaceutical Sciences, 18th edition, A. R. Gennaro, Ed., Mack Publishing Company [1990]; Pharmaceutical Formulation Development of Peptides and Proteins, S. Frokjaer and L. Hovgaard, Eds., Taylor & Francis [2000]; and Handbook of Pharmaceutical Excipients, 3rd edition, A. Kibbe, Ed., Pharmaceutical Press [2000]). The AT inhibitor of this invention preferably is formulated and administered as a sterile solution although it is within the scope of this invention to utilize lyophilized preparations. Sterile solutions are prepared by sterile filtration or by other methods known per se in the art. The solutions are then lyophilized or filled into pharmaceutical dosage containers. The pH of the solution generally is in the range of pH 3.0 to 9.5, e.g. pH 5.0 to 7.5. The protein or peptide typically is in a solution having a suitable pharmaceutically acceptable buffer, and the solution of protein may also contain a salt. In certain embodiment, detergent is added. For use in this invention AT inhibitor may be formulated into an injectable preparation. Parenteral formulations are suitable for use in the invention, preferably for intravenous administration. These formulations contain therapeutically effective amounts of AT inhibitor, are either sterile liquid solutions, liquid suspensions or lyophilized versions and optionally contain stabilizers or excipients. Typically, lyophilized compositions are reconstituted with suitable diluents, e.g. sterile water for injection, sterile saline and the like.

*Treatment of patients with a bleeding disorder with AT inhibitors*

AT inhibitor may be administered by injection intravascularly, or by other administration routes and/or sites. The dosage required depends amongst others on the clinical condition.

Above we have described the methodology to assess the effect of a varying degree of AT inhibition on thrombin and fibrin generation in hemophilic plasma in comparison with the effects of a dose-range of FVIII. In case of prophylactic treatment with AT-inhibitors, a similar degree of inhibition of AT is desired as is equivalent to the degree of inhibition that corresponds to supplementation with about 0.3 U FVIII as described under *"Assessment offunctional AT levels to guide the dose of AT inhibitor to be used in vivo"* hereinabove. Considering the pharmacokinetic properties of the AT inhibitor, the amount to be administered to yield a similar degree of AT inhibition in vivo as in the in vitro experiments, can be estimated. By monitoring levels of functional AT in the patient after administration of AT inhibitor, it is checked that the desired level of AT inhibition indeed occurs in the patient.

In case of an acute bleeding episode, AT inhibitor can be administered at slightly increasing doses and under guidance of functional levels of AT until bleeding stops. Treatment of patients with bleeding episodes due to other causes then hemophilia can be done using a similar strategy. AT inhibitor is administered to slowly decrease functional levels of AT until bleeding has ceased. Monitoring of levels of functional AT after administration of AT inhibitor is done with an assay as described hereinabove.

*Method for identifying a compound as an inhibitor of AT*

In a further aspect, the disclosure relates to a method for identifying as compound as an inhibitor of AT. The method preferably comprises the steps of: (a) adding the compound to pooled plasma from healthy volunteers; (b) mixing the pooled plasma with the compound as obtained in a) with at least one of isolated thrombin and isolated factor Xa; (c) determining the activity of at least one of thrombin and factor Xa in the mixture obtained in b) and comparing the activity in the mixture with the compound to the activity of at least one of thrombin and factor Xa mixed with the same plasma without the compound, wherein, preferably the activity of at least one of thrombin and factor Xa is determined in an assay using a chromogenic substrate; and, (d) identifying a compound that prevents a reduction in activity of at least one of thrombin and factor Xa in the plasma as compared to the same plasma without the compound, as an inhibitor of AT, whereby preferably the compound prevents a reduction in activity of at least one of thrombin and factor Xa of more than 50%. In preferred embodiment of the method, prior to step (a), the compound has been selected as a compound that specifically binds to AT. Preferably, in the method, the compound is a peptide, aptamer, antibody or antibody fragment that has been selected to specifically bind to AT; more preferably, the peptide, aptamer, antibody or antibody fragment has been selected to specifically bind to an epitope in the RSL sequence of AT.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

The following examples are offered for illustrative purposes only, and are not intended to limit the scope of the present invention as defined in the appended claims.

Description of the figures

Figure 1: Simplified scheme of coagulation. Activation starts with the exposure of blood to tissue factor (TF) which forms a complex with plasma FVII(a). The TF/FVIIa complex activates FX into FXa, which in its turn forms a complex with FV. The FXa/FVa complex converts the pro-enzym prothrombin into thrombin, the central enzyme of coagulation. This pathway can be considered as a basal pathway of coagulation. This basal pathway is amplified by two pathways, one consisting of FIX and FVIII, and another consisting of FXI. The FIX/FVIII pathway is activated by TF/FVIIa complex, which activates FIX into FIXa. FIXa in the FIXa/FVIIIa complex has proteolytic activity and can activate FX into FXa. Another amplification loop consists of FXI which upon activation by thrombin can also activate FIX. Not depicted in the figure are various clotting inhibitors including AT and phospholipid cofactor.

Figure 2: Structure of serpin A1 (Protein Data Bank (PDB) code 1QLP; PR Elliott et al., Nat.Struct Biol 1996;3:676-681). The reactive center loop (RCL) is at the top of the molecule. The dotted arrow indicates the "groove" in the central β-sheet in which the reactive center loop inserts.

Figure 3 Structural mechanism of the inhibitory action of a serpin with a protease. The structure shown is that of SERPINA1 Protein Data Bank (PDB) code 1QLP. Pathway b → c: Protease serpin interaction results in a stable covalent complex, in which the active site serine of the protease is connected to the PI-residue of the reactive site loop (= reactive center loop, RCL) of the serpin. As a result the protease hangs distorted and inactive at the base of the molecule. Pathway b → d: serpin-protease complex is dissociated before insertion of RSL into the central β-sheet is completed and conformational change in the active site of the serine protease is induced. As a result the active protease is released and an inactive serpin with stable conformation is left. In this situation the serpin behaves like a substrate for the protease, which escapes the conformational trap. References: PR Elliott et al., Nat.Struct Biol 1996;3:676-681; A Dementiev et al., J Biol Chem 2003;278:37881-37887; JA Huntington et al., Nature 2000;407:923-926; H Loebermann et al., J.Mol.Biol. 1984;177:531-557.

Figure 4 Cartoon depicting the mechanism of some AT inhibitors. Shown is the effect of a peptide inserting into the central β-sheet of AT or a monoclonal antibody (mAb) that prevent insertion of the RSL into the central β-sheet. Left part of the cartoon shows the normal situation: upon cleavage of the RSL by a target protease the cleaved RSL (residues P1-P14) insert into the central β-sheet thereby distorting the 3D-structure of the active site of the protease rendering it inactive. On the right, a peptide mimicking P1-P14 sequence of the RSL inserts into the central β-sheet thereby preventing insertion of AT's RSL. As an alternative inhibitor of AT, a mAb binding to the P4-P14 sequence of RSL prevents insertion of RSL into the central β-sheet rendering AT a substrate for its target proteases.

Figure 5 Affinity purified goat polyclonal antibodies against AT (anti-AT) completely inhibit the activity of AT in fresh human plasma as measured with thrombin. Affinity purified goat anti-AT antibodies (20 μg in 25 μl) were added to 25 μl of 1 to 10 diluted fresh human plasma, and incubated for 15 minutes at room temperature. 50 μl of the mixture was then incubated with thrombin (50 μl) and residual thrombin activity was measured by adding 50 μl of a solution of the chromogenic substrate Ethyl-Malonyl-S-Pro-Arg-pNA.AcOH (2mg/ml). The mixtures were then measured at 405nm every minute. The OD values are given on the Y-axis, time in minutes on the X-axis. Various concentrations of plasma were tested to generate a dose-response curve. Note that 100% plasma potently inhibits the activity of the added thrombin, and that addition of antibodies against AT (100% plasma + anti-AT) inhibits this effect of plasma completely.

Figure 6 Affinity purified goat polyclonal antibodies against AT (anti-AT) completely inhibit the activity of AT in fresh human plasma as measured with bovine FXa. Affinity purified goat anti-AT antibodies (6 μg in 7.5 μl) were added to 7.5 μl of 1 to 5 diluted fresh human plasma, and incubated for 15 minutes at room temperature. Then 15 μl with heparin (2.5 U/ml) were added and the mixtures were incubated for 30 min at 37°C. Then 75 μl of the chromogenic substrate MAPA-Gly-Arg-pNA (8.4 umol/ml) were added and the mixture was incubated for another 30 minutes. Finally, bovine FXa (75 μl) were added and residual FXa activity was measured at 405nm every minute. The OD values are given on the Y-axis, time in minutes on the X-axis. Various concentrations of plasma were tested to generate a dose-response curve. Note that 100% plasma potently inhibits the activity of the added FXa, and that addition of antibodies against AT (100% plasma + anti-AT) dose-dependently inhibits this effect of plasma.

Figure 7 Affinity purified goat polyclonal antibodies against AT boost thrombin generation in fresh human plasma. Purified goat anti-antithrombin III antibodies (a-AT) were added to fresh human plasma. The mixture was then incubated with tissue factor to induce activation of the coagulation system. Thrombin generation in the mixture was measured in real time with a chromogenic substrate, and expressed as nM by reference to a standard. Note the more than 2-fold increase in the amount of thrombin generated upon addition of the same amount of TF.

Figure 8 Affinity purified goat polyclonal antibodies against AT boost thrombin generation in plasma deficient for FVIII. Purified goat anti-antithrombin III antibodies (anti-AT) were added to the plasma sample. The mixture was then incubated with factor tissue factor to induce activation of the coagulation system. Thrombin in the mixture was measured with a chromogenic substrate. Note the more than 2-fold increase in the amount of thrombin generated in the sample when AT was fully inhibited (250 μg/ml).

## Examples

### Example 1: Inhibition of AT activity in plasma with polyclonal antibodies against AT

As an example to show that antibodies indeed can inhibit AT activity in human plasma, polyclonal antibodies were purified from serum of a goat immunized with human AT (Bioconnect, Huissen, the Netherlands; Catalog nr: AHP1750). Purified AT (Kybernin P, CSL-Behringwerke, Marburg, Germany; Catalog nr:83167111A) was coupled to CNBr-activated Sepharose 4B (GE Healthcare Life Sciences, Diegem, Belgium) according to manufacturer's instructions (approximately 25 mg AT to 1 gram CNBr-activated Sepharose). The beads were washed and subsequently incubated with 2.5 ml of goat antiserum against human AT diluted 1 to 2 in phosphate buffered saline, pH 7.4 (PBS), for 2 hours at room temperature. The beads were washed again and then eluted with 0.1 M glycine 0.15 NaCl, pH 2.5. The beads were spun down by centrifugation for 1 minute at 1300g, the supernatant was removed, immediately neutralized with 1M Tris, pH 8.5, and dialyzed against PBS. The resulting preparation was analyzed on SDS-PAGE for purity, and found to consist of one band with Mr of ~160.000 under non-reducing conditions, and of 2 bands with Mr ~50.000 and ~25.000 under reducing conditions.

Two assays for functional AT activity were used to evaluate the effects of the antibodies on functional AT in plasma. The first assay (STA ATIII kit, Catalog nr: 12145103140; Roche Diagnostics, Almere, the Netherlands) is a chromogenic assay using thrombin as the target protease. The principle of this assay is that a standard amount of thrombin is added to plasma in the presence of heparin, the plasma is then checked for residual thrombin activity using the chromogenic substrate Ethyl-Malonyl-S-Pro-Arg-pNA.AcOH (2mg/ml). In this assay 50 μL of 1 to 20 diluted (in diluent provided with the assay) plasma samples are tested. To assess their effects on AT function in plasma, 20 μg of the purified anti-AT antibodies in 25 μL were added to 25 μL of 1 to 10 diluted normal plasma. The mixture was then tested in the assay. The results clearly showed that the inhibitory effect of plasma on thrombin activity was neutralized upon addition of purified anti-AT antibodies (compare "100% plasma" with "100% plasma + anti-AT" and with "0% plasma" in Figure 5).

Though AT is the main inhibitor of thrombin in plasma, other inhibitors in plasma such as heparin cofactor II

may also contribute to the inhibition of thrombin. In contrast FXa is exclusively inhibited in plasma by AT. Therefore, the results with the anti-AT antibodies were confirmed in another assay (STA Rotachrom Heparin, Catalog nr: 11556959140; Roche Diagnostics), which is similar to the assay with thrombin described above except that bovine FXa is used as protease and Ethyl-Malonyl-S-Pro-Arg-pNA.AcOH as the chromogenic substrate (at 2 mg/ml). Furthermore 15 μL of 1 to 10 diluted (in diluent provided with the assay) plasma samples are tested. Mixtures of 6 μg of affinity purified anti-AT antibodies in 7.5 μL and 7.5 μL of 1 to 5 diluted normal plasma were made and tested in the assay. Addition of the affinity purified antibodies to fresh human plasma dose-dependently abandoned the inhibitory effect of plasma on FXa (compare "100% plasma" with "100% plasma + anti-AT" and with "0% plasma" in Figure 6).

The results shown in Figures 5 and 6 show that complete inhibition of AT activity in plasma can be achieved with polyclonal antibodies against AT. This effect of the affinity purified AT antibodies was confirmed in several other plasma samples: in all plasma samples tested complete inhibition of AT activity was observed upon addition of affinity purified polyclonal antibodies against AT.

Example 2: Effects of AT inhibitors on thrombin generation in normal and in FVIII-deficient plasma

In a next set of experiments we then established whether inhibition of AT by addition of the affinity purified anti-AT antibodies could affect thrombin generation induced by TF in normal plasma and in hemophilic plasma.

Thrombin is the key enzyme of the coagulation system and the generation of thrombin in time in vitro upon addition of TF to plasma is considered to be an excellent *ex vivo* model for coagulation. Thrombin generation in hemophilic plasma at low TF concentrations is severely impaired and therapeutic interventions that have been shown to be effective to stop bleeding in hemophilic patients, i.e. FVIII preparations, rFVIIa and the activated prothrombin complex such as FEIBA (J Astermark et al., Blood 2007;109:546-551; HR Roberts et al., Blood 2004;104:3858-3864) all have been shown to be able to restore thrombin generation to some extent (K Varadi et al., J Thromb Haemost 2003;1:2374-80; NM Aljamali et al., Haemophilia 2009;15:1318-26). Therefore, we selected this *ex vivo* model to evaluate the effects of AT inhibitors.

The model, the calibrated automated thrombogram assay was performed essentially as described by Hemker et al. (Pathophysiol Haemost Thromb 2002;32:249-2) and the manual provided by Thrombinoscope (Maastricht, the Netherlands). Coagulation was triggered by recalcification in the presence of 1 or 5 pM recombinant human tissue factor, 4 μM procoagulant phospholipids, and 417 μM fluorogenic substrate Z-Gly-Gly-Arg-AMC. Fluorescence was monitored with a fluorometer (Fluoroscan Ascent, ThermoLabsystems, Helsinki, Finland), and the endogenous thrombin potential (ETP) was calculated using the Thrombinoscope® software (Thrombinoscope). The resulting "Thrombogram" in platelet poor or platelet rich plasma measures hypocoagulability such as hemophilias or the use of anticoagulants but also hypercoagulabilties such as AT deficiency, and so on. As is shown in Figure 7, the addition of purified anti-AT antibodies induced an enhanced thrombin generation in normal plasma consistent with the existence of a hypercoagubility state, which is consistent with the inhibition of AT in the plasma.

The same experiment was performed except that FVIII-deficient plasma rather than pooled normal plasma was used. Patients with hemophilia lack factor VIII (or factor IX) and show much less thrombin generation at low TF concentration than healthy people. As can be seen in Figure 8, thrombin generation, as expected, was indeed reduced in hemophilic plasma with peak levels of approximately 20 nM, and consistent with a hypocoagulability of the plasma. Addition of affinity purified goat polyclonal antibodies against AT dose dependently boosted thrombin generation in the FVIII-deficient plasma. At the highest concentration added (250 μg/ml) a nearly 2-fold increase in thrombin generation was observed. As thrombin generation in plasma is considered to be an excellent indicator for the potential to generate thrombin in vivo, these data strongly support that inhibition of AT constitutes a therapeutic approach for hemophilia to restore the thrombin generation defect by bypassing FVIII.

## Claims

**1.** An inhibitor of antithrombin III for use in the treatment and prevention of bleeding:

i) in a subject suffering from an acquired or genetic bleeding disorder (hypocoagulability); or,
ii) in a subject having a clinical condition **characterised by** excessive bleeding, wherein the bleeding is not due to the use of anti-coagulants;

wherein the inhibitor is selected from the group consisting of:

a) a peptide or peptidomimetic that mimicks the amino acid sequence of RSL residues P1-P14;
b) an aptamer that specifically binds to antithrombin III; and,
c) an antibody or antibody fragment that specifically binds to antithrombin III,

and wherein the inhibitor, when added to pooled plasma from healthy volunteers, causes a decrease of functional antithrombin III levels by at least 20% as measured with a chromogenic assay for functional antithrombin III using factor Xa or thrombin.

**2.** An inhibitor for a use according to claim 1, wherein the bleeding disorder is haemophilia.

**3.** An inhibitor for a use according to claim 2, wherein the subject is a hemophiliac with neutralizing antibodies to factor VIII or factor IX.

**4.** An inhibitor for a use according to claim 3, wherein the inhibitor is used as prophylactic factor VIII or factor IX bypass therapy.

**5.** An inhibitor for a use according to claim 1, wherein the clinical condition **characterised by** excessive bleeding is one or more of surgery, trauma and internal bleeding.

**6.** An inhibitor for a use according to any one of claims 1 - 5, wherein the antibody or antibody fragment specifically binds to an epitope in the RSL sequence of antithrombin III.

**7.** An inhibitor for a use according to claim 6, wherein the antibody or antibody fragment impairs insertion of the RSL into the central β-sheet of antithrombin III.

**8.** An inhibitor for a use according to claim 6 or 7, wherein the epitope in the RSL sequence of antithrombin III comprises

one or more of the RSL residues P 1 - P14.

**9.** An inhibitor for a use according to claim 8, wherein the epitope comprises RSL residues around P10, RSL residues P5 - P10 or RSL residues P3 - P8.

## Patentansprüche

**1.** Inhibitor von Antithrombin III zur Verwendung bei der Behandlung und Vorbeugung von Blutung:

(i) in einem Subjekt, das an erworbener oder genetischer Blutgerinnungsstörung (Hypocoagulabilität) leidet, oder
(ii) in einem Subjekt, das sich in einem klinischen Zustand befindet, der durch übermäßige Blutung gekennzeichnet ist, wobei die Blutung nicht auf die Verwendung von gerinnungshemmenden Mitteln zurückzuführen ist,

wobei der Inhibitor ausgewählt ist aus der Gruppe bestehend aus:

a) Peptid oder Peptidomimetikum, das die Aminosäuresequenz von RSL-Resten P1-P14 nachahmt,
b) Aptamer, das spezifisch an Antithrombin III bindet, und
c) Antikörper oder Antikörperfragment, das spezifisch an Antithrombin III bindet,

und wobei der Inhibitor, wenn er dem gepoolten Plasma von gesunden Probanden zugesetzt wird, eine Abnahme des spiegels von funktionellem Antithrombin III um mindestens 20%, gemessen mit einem chromogenen Assay für funktionelles Antithrombin III unter Verwendung von Faktor Xa oder Thrombin, bewirkt.

**2.** Inhibitor für eine Verwendung gemäß Anspruch 1, wobei die Blutgerinnungsstörung Hämophilie ist.

**3.** Inhibitor für eine Verwendung gemäß Anspruch 2, wobei das Subjekt ein hämophiler Patient ist, der neutralisierende Antikörper gegen Faktor VIII oder Faktor IX aufweist.

**4.** Inhibitor für eine Verwendung gemäß Anspruch 3, wobei der Inhibitor in der prophylaktischen Faktor VIII- oder Faktor IX-Bypass-Behandlung verwendet wird.

**5.** Inhibitor für eine Verwendung gemäß Anspruch 1, wobei der klinische Zustand, der durch übermäßige Blutung gekennzeichnet ist, einer oder mehrere aus Operation, Trauma und innere Blutungen ist.

**6.** Inhibitor für eine Verwendung gemäß einem der Ansprüche 1 bis 5, wobei der Antikörper oder das Antikörperfragment spezifisch an ein Epitop in der RSL-Sequenz von Antithrombin III bindet.

**7.** Inhibitor für eine Verwendung gemäß Anspruch 6, wobei der Antikörper oder das Antikörperfragment die Einfügung des RSL in die zentrale □-Faltblattstruktur von Antithrombin III verschlechtert.

**8.** Inhibitor für eine Verwendung gemäß Anspruch 6 oder 7, wobei das Epitop in der RSL-Sequenz von Antithrombin III einen oder mehrere der RSL-Reste P1 - P14 umfasst.

**9.** Inhibitor für eine Verwendung gemäß Anspruch 8, wobei das Epitop RSL-Reste um P10 herum, RSL-Reste P5 - P10 oder RSL-Reste P3 - P8 umfasst.

## Revendications

**1.** Inhibiteur d'antithrombine III pour une utilisation lors du traitement et la prévention d'un saignement :

i) chez un sujet souffrant d'un trouble hémorragique acquis ou génétique (hypocoagulabilité) ; ou,
ii) chez un sujet présentant un état clinique **caractérisé par** un saignement excessif, le saignement n'étant pas dû à l'utilisation d'anticoagulants ;

dans lequel l'inhibiteur est choisi parmi le groupe constitué de :

a) un peptide ou peptidomimétique qui imite la séquence d'acides aminés des résidus P1-P14 de RSL ;
b) un aptamère qui se lie spécifiquement à l'antithrombine III ; et,
c) un anticorps ou un fragment d'anticorps qui se lie spécifiquement à l'antithrombine III,

et dans lequel l'inhibiteur, lorsqu'il est ajouté à du plasma combiné provenant de volontaires sains, provoque une diminution des niveaux d'antithrombine III fonctionnelle d'au moins 20 %, comme mesuré par un dosage chromogénique de l'antithrombine III fonctionnelle en utilisant le facteur Xa ou une thrombine.

**2.** Inhibiteur pour une utilisation selon la revendication 1, dans lequel le trouble hémorragique est l'hémophilie.

**3.** Inhibiteur pour une utilisation selon la revendication 2, dans lequel le sujet est un hémophile ayant des anticorps neutralisants dirigés contre le facteur VIII ou le facteur IX.

**4.** Inhibiteur pour une utilisation selon la revendication 3, dans lequel l'inhibiteur est utilisé comme thérapie prophylactique court-circuitant le facteur VIII ou le facteur IX.

**5.** Inhibiteur pour une utilisation selon la revendication 1, dans lequel l'état clinique **caractérisé par** un saignement excessif est l'un ou plusieurs parmi une intervention chirurgicale, un traumatisme et une hémorragie interne.

**6.** Inhibiteur pour une utilisation selon l'une quelconque des revendications 1-5, dans lequel l'anticorps ou le fragment d'anticorps se lie spécifiquement à un épitope dans la séquence RSL de l'antithrombine III.

**7.** Inhibiteur pour une utilisation selon la revendication 6, dans lequel l'anticorps ou le fragment d'anticorps pertube l'insertion de la RSL dans la feuille-β centrale de l'antithrombine III.

**8.** Inhibiteur pour une utilisation selon la revendication 6 ou 7, dans lequel l'épitope dans la séquence RSL de l'antithrombine III comprend un ou plusieurs des résidus Pl-P 14 de RSL.

**9.** Inhibiteur pour une utilisation selon la revendication 8, dans lequel l'épitope comprend des résidus de RSL autour de P10, des résidus P5-P10 de RSL ou des résidus P3-P8 de RSL.

*Fig. 1*

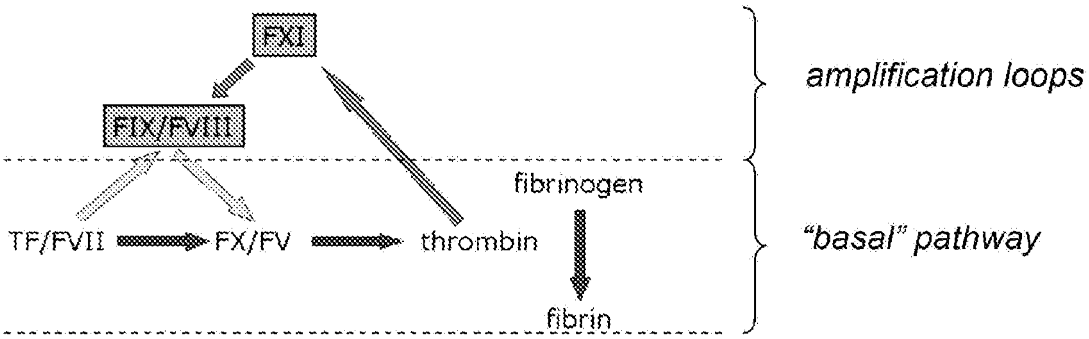
FXI
FIX/FVIII
TF/FVII
FX/FV
thrombin
fibrinogen
fibrin
amplification loops
"basal" pathway

*Fig. 2*

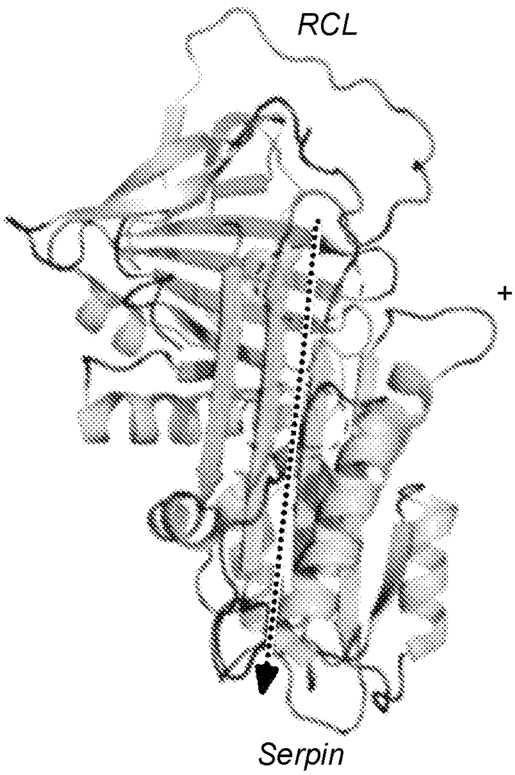
RCL
+
Serpin

Fig. 3

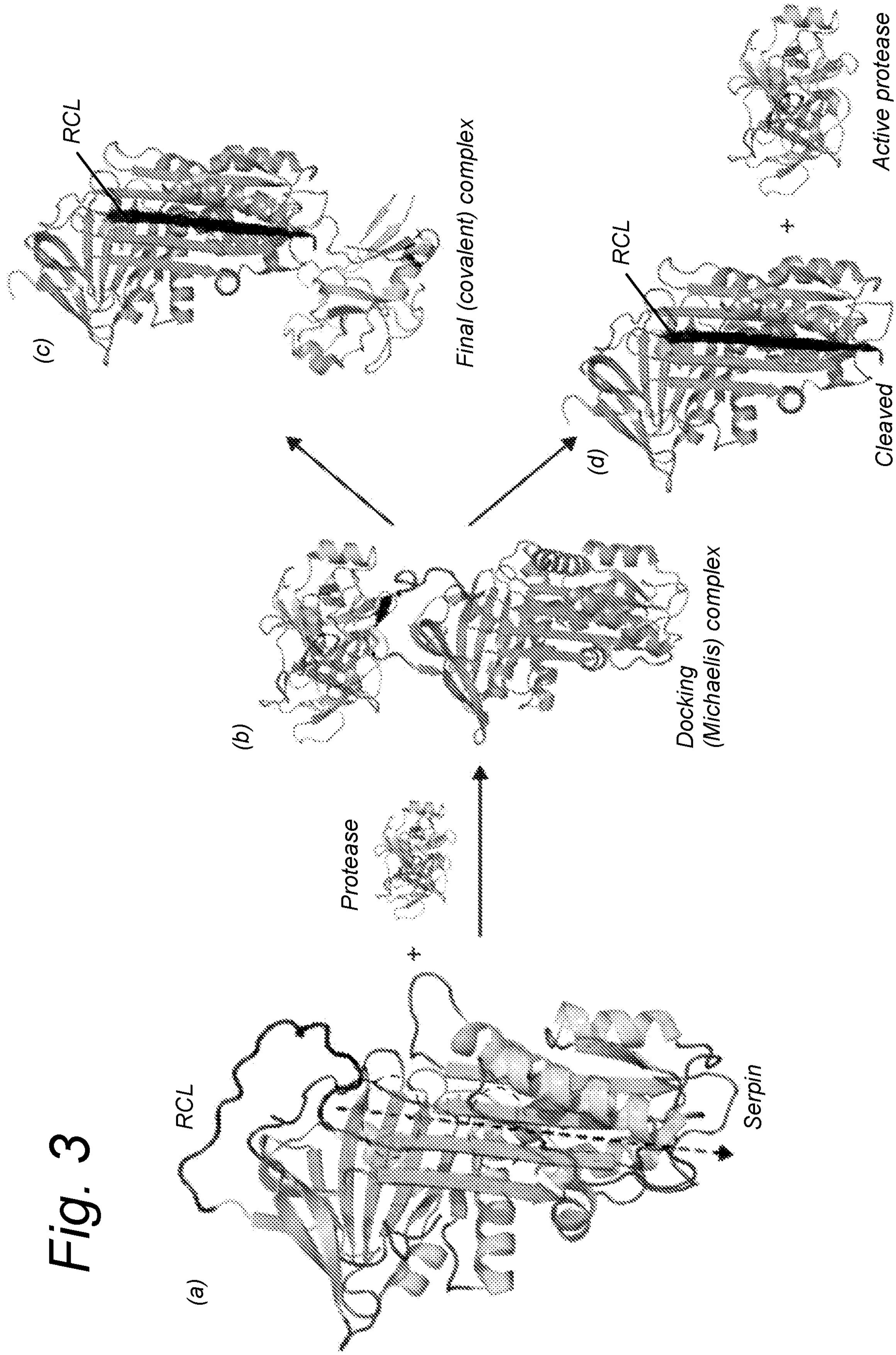
(a)
RCL
Serpin
Protease
(b)
Docking
(Michaelis) complex
(c)
RCL
Final (covalent) complex
(d)
RCL
Cleaved
Serpin
Active protease

*Fig. 4*

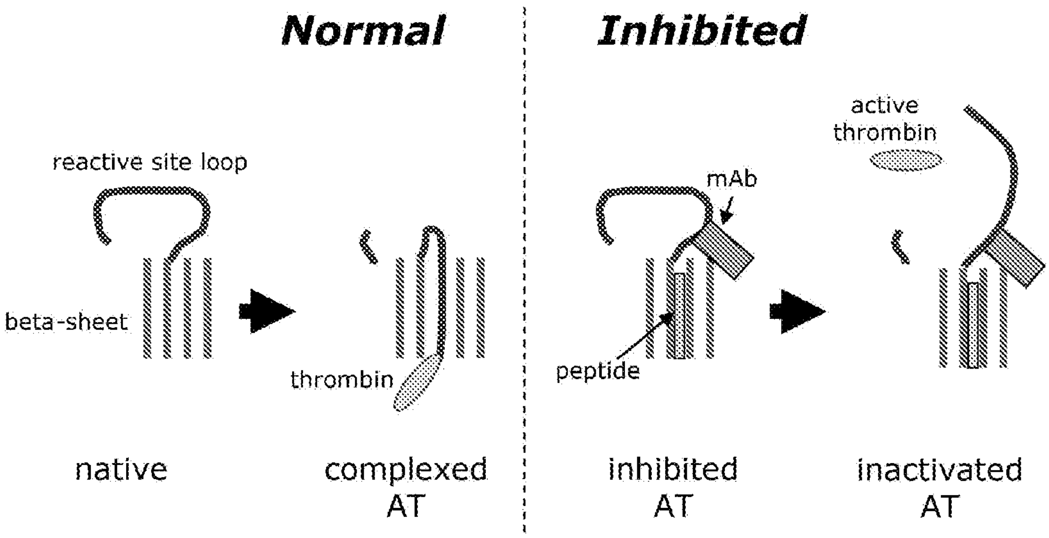
Normal
Inhibited
reactive site loop
beta-sheet
thrombin
active
thrombin
mAb
peptide
native
complexed
AT
inhibited
AT
inactivated
AT

# Fig. 5

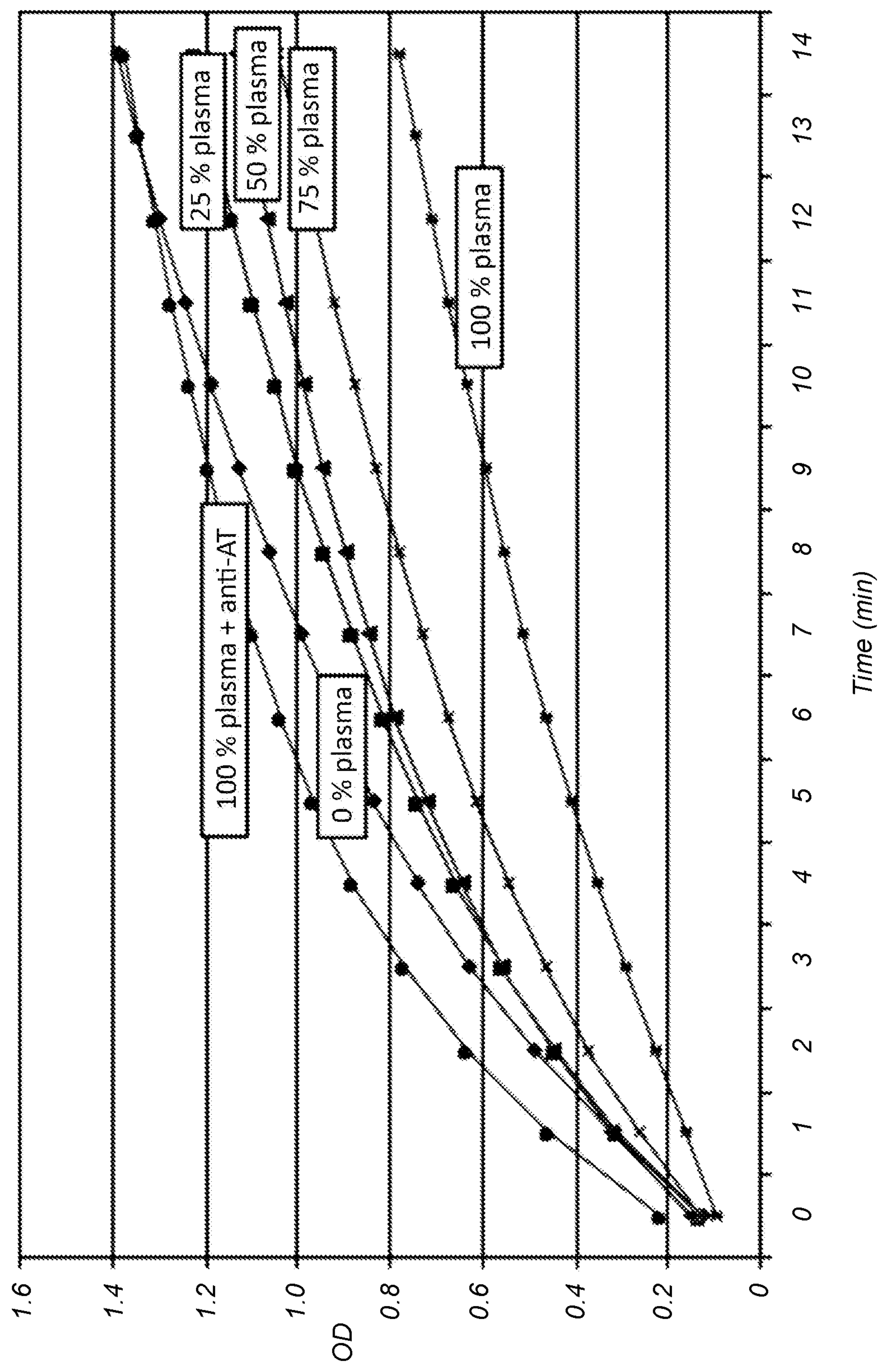
25 % plasma
50 % plasma
75 % plasma
100 % plasma
100 % plasma + anti-AT
0 % plasma
OD
Time (min)

Fig. 6

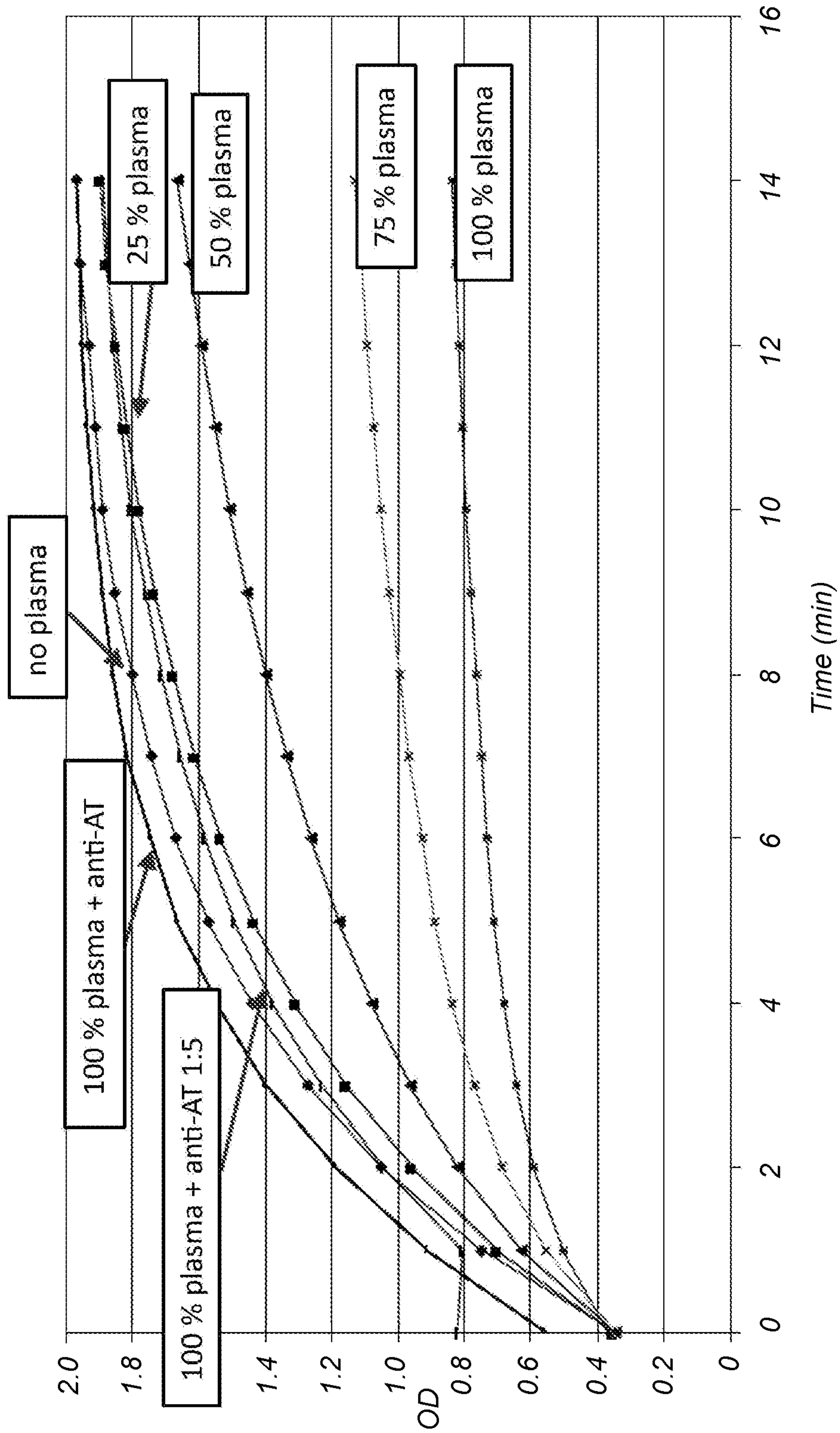
no plasma
100 % plasma + anti-AT
100 % plasma + anti-AT 1:5
25 % plasma
50 % plasma
75 % plasma
100 % plasma
OD
2.0
1.8
1.6
1.4
1.2
1.0
0.8
0.6
0.4
0.2
0
0
2
4
6
8
10
12
14
16
Time (min)

*Fig. 7*

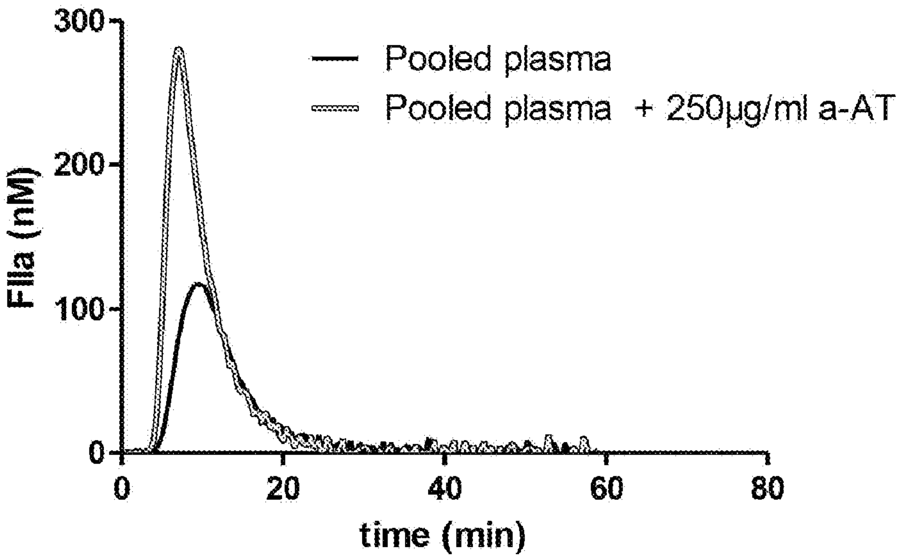
300
200
100
0
FIIa (nM)
0
20
40
60
80
time (min)
Pooled plasma
Pooled plasma + 250µg/ml a-AT

*Fig. 8*

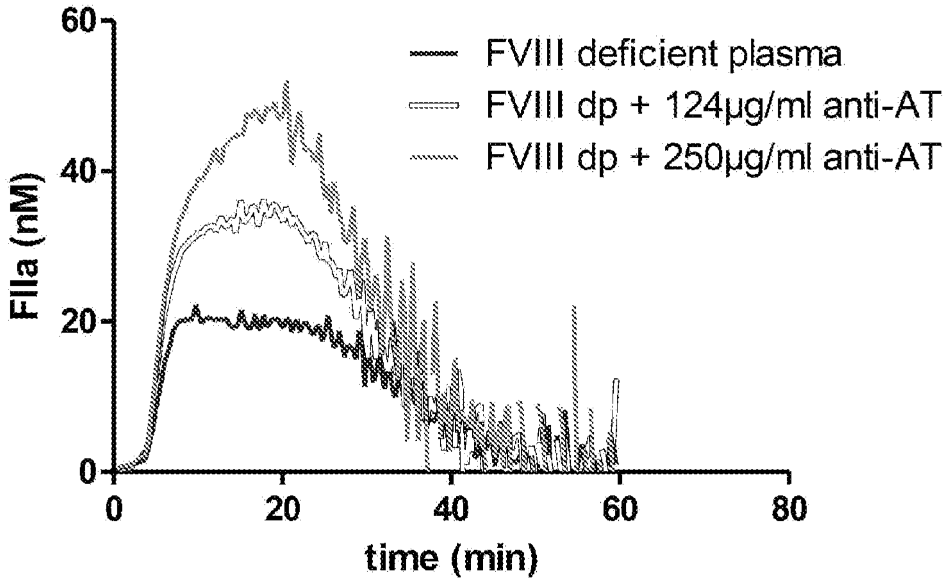
60
40
20
0
FIIa (nM)
0
20
40
60
80
time (min)
FVIII deficient plasma
FVIII dp + 124µg/ml anti-AT
FVIII dp + 250µg/ml anti-AT

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- NL 2012050581 W
- US 61527140 B


### Non-patent literature cited in the description


- **MANNUCCI et al.** *N Engl J Med,* 2001, vol. 344, 1773-1779
- **GOUDEMOND et al.** *Blood,* 2006, vol. 107, 46-51
- **GOUW et al.** *Blood,* 2007, vol. 109, 4693-4697
- **J ASTERMARK et al.** *Blood,* 2007, vol. 109, 546-551
- **HR ROBERTS et al.** *Blood,* 2004, vol. 104, 3858-3864
- **WK HOOTS.** *Blood,* 2007, vol. 109, 395-396
- **MANCO-JOHNSON et al.** *N Engl J Med,* 2007, vol. 357, 535-44
- **O'CONNEL et al.** *JAMA,* 2006, vol. 295, 293-298
- **LEVI et al.** *N Engl J Med,* 2010, vol. 363, 1791-1800
- **ISHIGURO et al.** *J Clin Invest.,* 2000, vol. 106, 873-878
- **PS MCLEAN et al.** *Drugs,* 2007, vol. 67, 1429-1440
- **VAN 'T VEER et al.** *J Biol Chem.,* 1997, vol. 272, 4367-4377
- **RUBY HP LAW et al.** *Genome Biology,* 2006, vol. 7, 216.1-216.11
- **BEECK et al.** *Blood Coagulation Fibrinolysis,* 2000, vol. 11, 127-35
- **I BJORK et al.** *J.Biol.Chem.,* 1992, vol. 267, 1976-1982
- **S HE et al.** *J.Immunol.,* 1996, vol. 154, 2009-2013
- **S HE et al.** *Molecular Medicine,* 1998, vol. 4, 119-128
- **S ASAKURA et al.** *J Biol Chem,* 1990, vol. 265, 5135-6
- **P HERION et al.** *Blood,* 1985, vol. 65, 1201-7
- **S KNOLLER et al.** *Eur J Bioch,* 1989, vol. 180, 319-27
- **HARLOW et al.** Antibodies: A Laboratory Manual. Cold Spring Harbor Publications, 1988, 726
- **CAMPBELL, A.M.** Monoclonal Antibody Technology Techniques in Biochemistry and Molecular Biology. Elsevier Science Publishers, 1984
- **ST. GROTH et al.** *J. Immunol. Methods,* 1980, vol. 35, 1-21
- **HACK et al.** *J Immunol.,* 1988, vol. 141, 1602-9
- **MJ MANCO-JOHNSON et al.** *N Engl J Med,* 2007, vol. 357, 535-44
- **J GOUDEMOND et al.** *Blood,* 2006, vol. 107, 46-51
- **SC GOUW et al.** *Blood,* 2007, vol. 109, 4693-4697
- **SA MAYER et al.** *N.Engl.J.Med.,* 2005, vol. 352, 777-85
- **SA MAYER et al.** *N.Engl.J.Med.,* 2008, vol. 358, 2127-37
- Remington's Pharmaceutical Sciences. Mack Publishing Company, 1990
- Pharmaceutical Formulation Development of Peptides and Proteins. Taylor & Francis, 2000
- Handbook of Pharmaceutical Excipients. Pharmaceutical Press, 2000
- **PR ELLIOTT et al.** *Nat.Struct Biol,* 1996, vol. 3, 676-681
- **A DEMENTIEV et al.** *J Biol Chem,* 2003, vol. 278, 37881-37887
- **JA HUNTINGTON et al.** *Nature,* 2000, vol. 407, 923-926
- **H LOEBERMANN et al.** *J.Mol.Biol.,* 1984, vol. 177, 531-557
- **K VARADI et al.** *J Thromb Haemost,* 2003, vol. 1, 2374-80
- **NM ALJAMALI et al.** *Haemophilia,* 2009, vol. 15, 1318-26
- **HEMKER et al.** *Pathophysiol Haemost Thromb,* 2002, vol. 32, 249-2

SEQUENCE LISTING

<110> UMC Utrecht Holding B.V.

<120> Compounds for use in boosting coagulation

<130> P6041926PCT

<140> PCT/NL2012/050581
<141> 2012-08-24

<150> US 61/527,140
<151> 2011-08-25

<160> 1

<170> PatentIn version 3.3

<210> 1
<211> 14
<212> PRT
<213> Artificial

<220>
<223> the amino acid sequence of RSL residues P1-P14

<400> 1

```
Ser Glu Ala Ala Ala Ser Thr Ala Val Val Ile Ala Gly Arg
1               5                   10
```